(19) Europäisches Patentamt
European Patent Office
Office européen des brevets

(11) EP 2 124 869 B1

(12) **FASCICULE DE BREVET EUROPEEN**

(45) Date de publication et mention de la délivrance du brevet:
**15.08.2012 Bulletin 2012/33**

(21) Numéro de dépôt: **08762065.4**

(22) Date de dépôt: **12.02.2008**

(51) Int Cl.:
***A61K 8/04*** *(2006.01)* ***A61K 8/81*** *(2006.01)*
***A61K 8/90*** *(2006.01)* ***A61Q 5/00*** *(2006.01)*

(86) Numéro de dépôt international:
**PCT/FR2008/050213**

(87) Numéro de publication internationale:
**WO 2008/104698 (04.09.2008 Gazette 2008/36)**

# (54) DISPERSION DE PARTICULES DE POLYMÈRE, COMPOSITION LA COMPRENANT ET PROCÉDÉ DE TRAITEMENT COSMÉTIQUE

POYLMERPARTIKELDISPERSION, ZUSAMMENSETZUNG DAMIT UND KOSMETISCHES BEHANDLUNGSVERFAHREN

POLYMER PARTICLE DISPERSION, COMPOSITION CONTAINING THE SAME AND COSMETIC TREATMENT METHOD

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **19.02.2007 FR 0753336**
**01.03.2007 US 904175 P**

(43) Date de publication de la demande:
**02.12.2009 Bulletin 2009/49**

(73) Titulaire: **L'Oréal**
**75008 Paris (FR)**

(72) Inventeurs:
- **MOUGIN, Nathalie**
  **F-75011 Paris (FR)**
- **JEGOU, Gwenaëlle**
  **F-91240 Saint Michel Sur Orge (FR)**

(74) Mandataire: **Dodin, Catherine**
**L'Oréal**
**D.I.P.I.**
**25-29 Quai Aulagnier**
**92600 Asnières (FR)**

(56) Documents cités:
**EP-A- 1 600 146** **EP-A- 1 772 477**
**WO-A-2006/005822** **WO-A-2007/003784**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 124 869 B1

Printed by Jouve, 75001 PARIS (FR)

## Description

La présente invention a trait à une nouvelle dispersion de particules de polymères bien particuliers, dispersées dans un milieu non aqueux, ainsi qu'aux compositions notamment cosmétiques ou pharmaceutiques comprenant ladite dispersion.

Parmi les polymères employés dans le domaine cosmétique, et plus particulièrement dans le domaine capillaire, on peut citer les polymères cationiques tels que ceux à base de chlorure de dimethyldiallylammonium, notamment connus pour protéger et/ou embellir le cheveu, grâce à leur forte substantivité. Toutefois, on n'observe aucun effet de mise en forme des cheveux lors de l'emploi de ce type de polymères. Par ailleurs, leur incompatibilité avec la plupart des agents propulseurs ne permet pas de les utiliser dans des produits aérosol tels que les laques.

On connaît par EP1323753, des compositions capillaires comprenant des dispersions aqueuses de polymères hydrophobes cationiques qui apportent des propriétés coiffantes, par exemple lorsqu'ils sont employés dans les shampoings. Toutefois, ces compositions ne présentent pas une très bonne cosmétique en milieu humide, notamment au fur et à mesure des applications des shampooings.

Afin d'obtenir un bon effet coiffant tout en conservant aux compositions des propriétés cosmétiques acceptables, il a été proposé des polymères possédant des propriétés de coiffage, véhiculés dans un solvant cosmétique. Ainsi, dans les documents WO91/15185 et WO98/18433, il est proposé des compositions cosmétiques comprenant des polymères hydrophobes ou non hydrosolubles, véhiculés en solution dans un solvant organique. Toutefois, la nécessité d'employer des polymères solubles dans un milieu organique, implique une faible variabilité des structures chimiques disponibles.

Par ailleurs, on a constaté que les solutions organiques de polymères hydrophobes présentent généralement une viscosité importante, liée à la teneur en polymère de la solution, ce qui rend difficile la formulation ultérieure de ces polymères et de leurs solutions.

Il est par ailleurs connu d'utiliser en cosmétique des dispersions de particules de polymère, généralement de taille nanométrique, dans des milieux organiques, et notamment, par EP749747, des dispersions non aqueuse de particules de polyacrylate ou polyméthacrylate de méthyle, dans une huile de paraffine non volatile ou dans l'isododécane, par exemple. Toutefois, ces dispersions ne permettent pas d'obtenir des propriétés cosmétiques satisfaisantes, notamment en terme d'effet coiffant et de démêlage. Par ailleurs, le toucher des compositions les comprenant peut se révéler peu satisfaisant, notamment quant à la douceur.

La demanderesse a découvert de manière surprenante de nouvelles dispersions de particules de polymères, stabilisées par des stabilisants, dans des milieux non aqueux, qui permettent d'apporter les propriétés cosmétiques recherchées (toucher, douceur, démêlage), tout en améliorant les propriétés de coiffant.

La présence de monomères cationiques dans le polymère en dispersion permet notamment d'apporter plus de douceur, et d'améliorer le démêlage; le dépôt sur le cheveu est plus efficace, ce qui est particulièrement avantageux dans le cas des cheveux abîmés. Par ailleurs, la présence de monomères cationiques permet d'améliorer la tenue de la boucle, c'est-à-dire l'effet coiffant.

L'invention a donc pour objet une dispersion de particules d'au moins un polymère éthylénique stabilisé en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés; caractérisée par le fait que ledit polymère éthylénique comprend de 50 à 100% en poids de monomère hydrophile cationique, ayant un log p inférieur ou égal à 2, ou d'un mélange de tels monomères, par rapport au poids total de monomères.

L'invention a également pour objet une composition cosmétique ou pharmaceutique comprenant, dans un milieu cosmétiquement ou pharmaceutiquement acceptable, au moins une dispersion telle que définie ci-dessus.

Les dispersions selon l'invention permettent d'obtenir un niveau de soin et de démêlage suffisant, supérieur à celui de l'art antérieur, et durable dans le temps. L'invention permet de préparer des polymères aisément véhiculables, étant donné que les dispersions ont des viscosités peu élevées, ce qui facilite leur mise en oeuvre dans les compositions cosmétiques.

De plus, ces compositions permettent de conférer du volume et de la tenue à la chevelure.

En outre, ces dispersions ou compositions les comprenant apportent des propriétés intéressantes, en particulier en mode rincé. Elles permettent d'obtenir, en plus des effets coiffants et de la tenue, un toucher doux et non collant, une bonne douceur, ainsi qu'une facilité de démêlage du cheveu, en milieu sec et/ou humide.

Ces polymères peuvent être également utilisés dans des produits dits "non rincés" de type produit de soin du cheveu (masque capillaire), laque ou gels/mousses coiffants, pour apporter, outre du coiffant, de la cosmétique à la chevelure (toucher, douceur, lissage, démêlage). Ils peuvent également être introduits dans des compositions de pré- ou de post-traitement après coloration capillaire, permanente ou défrisage.

Un autre avantage de l'invention réside dans le fait que les particules de polymère peuvent être de très petite taille, notamment nanométrique, ce qui n'est pas le cas avec, par exemple, d'autres types de particules telles que des microsphères dont le diamètre est généralement supérieur à 1 micron. Or, une taille importante de l'ordre du micron a

pour inconvénient d'entraîner une certaine visibilité des particules à l'oeil, lorsqu'elles sont dans une composition et lorsqu'elles sont appliquées sur la peau, ainsi qu'une mauvaise stabilité de la composition, notamment dans le temps.

Ainsi, les dispersions selon l'invention permettent l'obtention de compositions stables, qui peuvent en outre être transparentes, translucides ou opaques, au choix, selon la taille des particules de polymère qui y sont dispersées.

Les dispersions selon l'invention sont donc constituées de particules, généralement sphériques, d'au moins un polymère éthylénique, stabilisé en surface par un stabilisant, dans un milieu non aqueux.

Les dispersions selon l'invention peuvent notamment se présenter sous forme de nanoparticules de polymères en dispersion stable dans un milieu non aqueux. Les nanoparticules sont de préférence d'une taille comprise entre 5 et 600 nm, notamment 10 à 500 nm, encore mieux 15 à 450 nm, étant donné qu'au-delà d'environ 600 nm, les dispersions de particules deviennent beaucoup moins stables. Notamment, ces particules restent à l'état de particules élémentaires, sans former d'agglomérats, lorsqu'elles sont en dispersion dans lesdits milieux non aqueux.

Par polymère "éthylénique", on entend un polymère obtenu par polymérisation d'au moins 2 monomères, identiques ou différents, comprenant une insaturation éthylénique.

Ledit polymère éthylénique peut être choisi par l'homme du métier en fonction de ses propriétés, selon l'application ultérieure souhaitée pour la composition. Ces polymères peuvent être en particulier réticulés.

Les polymères selon l'invention peuvent être des homopolymères ou des copolymères linéaires, branchés, greffés, ou même en étoiles. Ils peuvent être statistiques ou alternés. De préférence, ce sont des copolymères statistiques linéaires.

Le polymère éthylénique selon l'invention comprend de 50 à 100% en poids de monomère cationique hydrophile, ou d'un mélange de tels monomères, par rapport au poids total de monomères. Il comprend de préférence de 51 à 99,5% en poids, mieux de 52 à 99% en poids, voire de 55 à 98% en poids, notamment de 60 à 95% en poids, préférentiellement de 65 à 85% en poids de monomère cationique hydrophile, seul ou en mélange, par rapport au poids total de monomères initiaux.

Les monomères cationiques hydrophiles sont de préférence monofonctionnels, c'est-à-dire qu'ils ne comportent de préférence qu'une seule fonction polymérisable, notamment vinylique.

Par "monomère cationique", on entend tout monomère possédant une charge lorsqu'il est placé en milieu aqueux, à un pH compris entre 3 et 12.

Par "monomère hydrophile", on entend au sens de la présente invention un monomère ayant une valeur du logarithme du coefficient de partage apparent octanol-1/eau, aussi appelé log p, inférieure ou égale à 2, par exemple comprise entre -10 et 2, de préférence comprise entre -5 et 1,9, et notamment entre -2,5 et 1,5.

Les valeurs de log p sont connues et sont déterminées selon un test standard qui détermine la concentration du monomère dans l'octanol-1 et l'eau.

Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional référence book, 1995). Il existe encore un site Internet qui fournit des valeurs estimées (adresse : http://esc.syrres.com/interkow/kowdemo.htm).

Parmi les monomères cationiques hydrophiles susceptibles d'être employés dans le cadre de l'invention, on peut citer, seul ou en mélange, les monomères de formules (I) et (II) suivantes, ainsi que leurs sels:

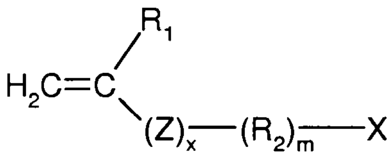

(I)

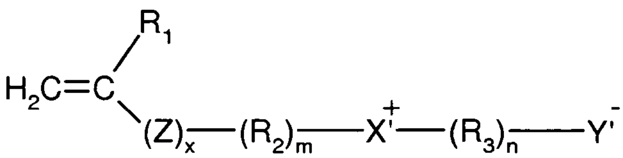

(II)

dans lesquelles :

- R1 est un atome d'hydrogène ou un radical hydrocarboné, linéaire ou ramifié, de type $C_pH_{2p+1}$, avec p étant un entier compris entre 1 et 12, inclus; Notamment, R1 peut représenter un radical méthyle, éthyle, propyle, butyle. De préférence, R1 représente l'hydrogène ou un radical méthyle.

- Z est un groupement divalent choisi parmi -COO-, -CONH-, $-CONCH_3-$, -OCO-, - O-, $-SO_2-$ -CO-O-CO- ou -CO-

$CH_2$-CO-; de préférence, Z est choisi parmi COO et CONH.

- x est 0 ou 1, de préférence 1.
- R2 et R3 sont, indépendamment l'un de l'autre, un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;

Dans les radicaux R2 et/ou R3, le ou les hétéroatomes, quand ils sont présents, peuvent être intercalés dans la chaîne dudit radical R2/R3, ou bien ledit radical R2/R3 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle). Notamment R2 et/ou R3 peut être :

- un radical alkylène ayant 1 à 20 atomes de carbone, tel que méthylène, éthylène, n-propylène, isopropylène, n-butylène, isobutylène, tertiobutylène, pentylène, isopentylène, n-hexylène, isohexylène, heptylène, isoheptylène, n-octylène, isooctylène, nonylène, isononylène, décylène, isodécylène, n-dodécylène, isododécylène, tridécylène, n-tétradécylène, hexadécylène, n-octadécylène, docosanylène, arachinylène;
- un radical cycloalkylène ayant 5 à 10 atomes de carbone, substitué ou non, tel que cyclopentylène, cyclohexylène, cycloheptylène, cyclooctylne, cyclononylène, cyclodécylène;
- un radical phénylène $-C_6H_4-$(ortho, méta ou para) éventuellement substitué, par un radical alkyle en C1-C12 comprenant éventuellement 1 à 25 hétéroatomes choisis parmi N, O, S, F, Si et/ou P; ou
- un radical benzylène $-C_6H_4-CH_2-$ éventuellement substitué, par un radical alkyle en C1-C12 comprenant éventuellement 1 à 25 hétéroatomes choisis parmi O, N, S, F, Si et P;
- ou un mélange de ces radicaux;
- m est 0 ou 1 ; de préférence 1 ;
- n est compris entre 1 et 100, de préférence 1 et 5 inclus;
- X est :

(a) un groupe guanidino ou un groupe amidino; ou

(b) un groupe de formule $-N(R_6)(R_7)$ avec R6 et R7 représentant, indépendamment l'un de l'autre, (i) un atome d'hydrogène, (ii) un groupement alkyle linéaire, ramifié ou cyclique, saturé ou insaturé, éventuellement aromatique, comprenant de 1 à 18 atomes de carbone, notamment 1 à 6 atomes de carbone, pouvant comprendre 1 à 10 hétéroatomes choisis parmi O, N, S, F, Si et P; (iii) R6 et R7 pouvant former avec l'atome d'azote un cycle de formule :

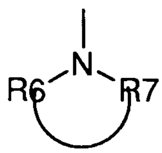

saturé ou insaturé, éventuellement aromatique, comprenant au total 5 à 8 atomes, et notamment 3 à 7 atomes de carbone et/ou 1 à 4 hétéroatomes choisi parmi O, S et N; ledit premier cycle pouvant être fusionné avec un ou plusieurs autres cycles, saturés ou insaturés, éventuellement aromatiques, comprenant chacun 5 à 7 atomes, et notamment 4 à 7 atomes de carbone et/ou 2 à 4 hétéroatomes choisi parmi O, S et N.

Par exemple, R6 et R7 peuvent être choisis parmi l'hydrogène, un groupement méthyle, éthyle, propyle, isopropyle, n-butyle, t-butyle, isobutyle, octyle, lauryle, stéaryle.

De préférence R6 et R7 sont choisis, indépendamment l'un de l'autre, parmi H, CH3 et C2H5.

(c) un cycle :

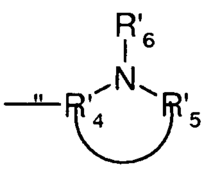

dans lequel R'4 et R'5 forment avec l'atome d'azote un cycle, saturé ou insaturé, éventuellement aromatique, comprenant

au total 5 à 8 atomes, et notamment 3 à 7 atomes de carbone et/ou 1 à 4 hétéroatomes choisi parmi O, S et N; ledit cycle pouvant être fusionné avec un ou plusieurs autres cycles, saturés ou insaturés, éventuellement aromatiques, comprenant chacun 5 à 7 atomes, et notamment 4 à 7 atomes de carbone et/ou 2 à 4 hétéroatomes choisi parmi O, S et N; et R'6 est choisi parmi H, $-CH_3$ et $-C_2H_5$.

Par exemple, X peut constituer un cycle aromatique ou non comportant un groupement amine tertiaire ou peut représenter un hétérocycle aromatique ou non, contenant un azote tertiaire. On peut citer les radicaux de type pyridinyle, indolyle, isoindolinyle, imidazolyle, imidazolinyle, piperidinyl, piperazinyl, pyrazolyl, pyrazolinyl, quinoline, pyrrolidinyl, quinidinyl, morpholine, et leurs mélanges.

De préférence, X est :

- un groupe de formule $-N(R_6)(R_7)$ avec R6 et R7 représentant, indépendamment l'un de l'autre, (i) un atome d'hydrogène, (ii) un groupement alkyle linéaire, ramifié ou cyclique, saturé ou insaturé, éventuellement aromatique, comprenant de 1 à 18 atomes de carbone, notamment 1 à 6 atomes de carbone (iii) R6 et R7 pouvant former avec l'atome d'azote un cycle de formule :

  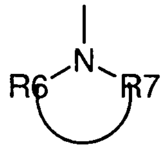

  saturé ou insaturé, éventuellement aromatique, comprenant au total 5 à 7 atomes, et notamment 4 à 6 atomes de carbone et éventuellement 1 à 3 hétéroatomes choisi parmi O, S et N;
- un cycle :

  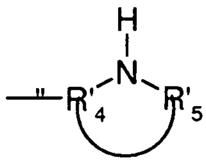

  dans lequel R'4 et R'5 forment avec l'atome d'azote un cycle, saturé ou insaturé, éventuellement aromatique, comprenant 5 à 7 atomes, notamment 4 à 6 atomes de carbone.

Préférentiellement, X est un groupe $NH_2$, $N(CH_3)_2$, pyridinyle, imidazolyle, piperidinyl, piperazinyl ou morpholine.

- X'+ est un groupe divalent de formule $-N^+(R_6)(R_7)-$ avec R6 et R7 représentant, indépendamment l'un de l'autre, (i) un atome d'hydrogène, (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; (iii) R6 et R7 pouvant former avec l'atome d'azote un cycle de formule :

  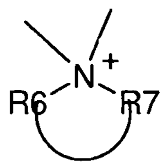

  saturé ou insaturé, éventuellement aromatique, comprenant au total 5 à 8 atomes, et notamment 3 à 7 atomes de carbone et/ou 1 à 4 hétéroatomes choisi parmi O, S et N; ledit premier cycle pouvant être fusionné avec un ou plusieurs autres cycles, saturés ou insaturés, éventuellement aromatiques, comprenant chacun 5 à 7 atomes, et notamment 4 à 7 atomes de carbone et/ou 2 à 4 hétéroatomes choisi parmi O, S et N.

Par exemple, R6 et R7 peuvent être choisis parmi l'hydrogène, un groupement méthyle, éthyle, propyle, isopropyle, n-butyle, t-butyle, isobutyle, octyle, lauryle, stéaryle. De préférence R6 et R7 sont choisis, indépendamment l'un de l'autre, parmi H, CH3 et C2H5.

- X'+ peut également être un cycle divalent de formule :

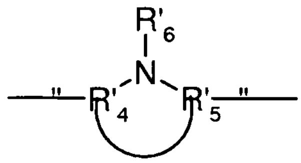

dans lequel R'4 et R'5 forment avec l'atome d'azote un cycle, saturé ou insaturé, éventuellement aromatique, comprenant au total 5 à 8 atomes, et notamment 3 à 7 atomes de carbone et/ou 1 à 4 hétéroatomes choisi parmi O, S et N; ledit cycle pouvant être fusionné avec un ou plusieurs autres cycles, saturés ou insaturés, éventuellement aromatiques, comprenant chacun 5 à 7 atomes, et notamment 4 à 7 atomes de carbone et/ou 2 à 4 hétéroatomes choisi parmi O, S et N; et R'6 est choisi parmi H, $-CH_3$ et $-C_2H_5$.

Parmi les radicaux $X'^{+}$ préférés, on peut citer les radicaux divalents de type pyridinyle, imidazolyle, piperidinyl, piperazinyl, pyrrolidinyl, morpholine, et leurs mélanges; et plus particulièrement les radicaux divalents pyridinyle, imidazolyle et piperazinyle.

- $Y'^{-}$ est un groupement choisi parmi $-COO^{-}$, $-SO_3^{-}$, $-OSO_3^{-}$, $-PO_3^{2-}$ et $-OPO_3^{2-}$.

Parmi les monomères de formule (I) préférés, on peut citer, seul ou en mélange :

- le (méth)acrylamide de diméthylaminopropyle (-0,343 et 0,210), le (méth)acrylamide de diméthylaminoéthyle (-0,250 et 0,302), le (méth)acrylamide de diéthylaminopropyle (0,813);
- le (méth)acrylate de diéthylaminoéthyle, le (méth)acrylate de diméthylaminoéthyle (0,948 et 1,5), le (méth)acrylate de morpholinoéthyle (0,554); le (méth)acrylate de tert-butylaminoéthyle;
- la vinylimidazole (0,96), la vinylpyridine (1,20), la vinylamine,
  et les monomères ci-après :

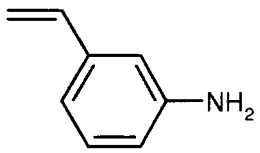

(1,418)

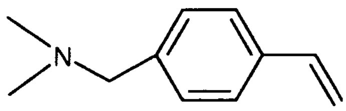

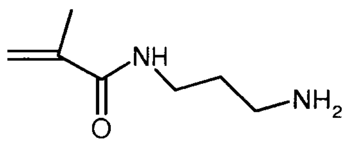

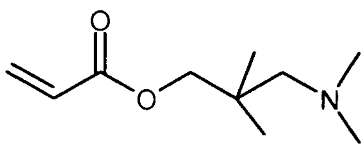

(1,861)

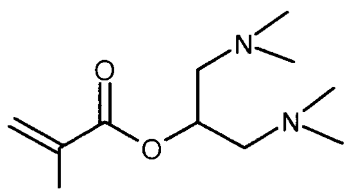

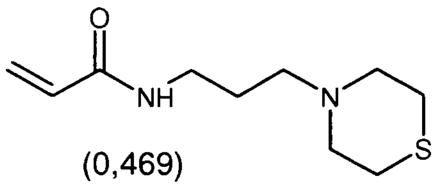

(0,469)

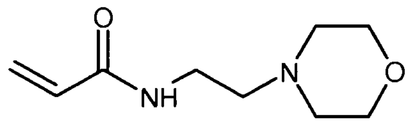

(-0,52)

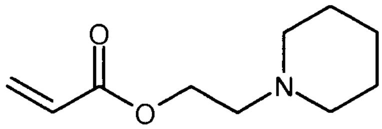

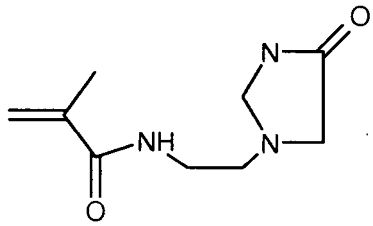

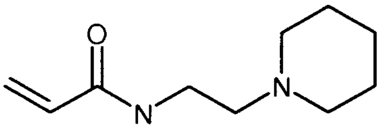

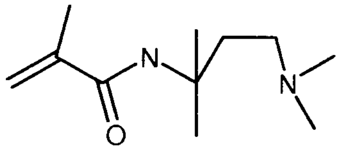

(0.906)

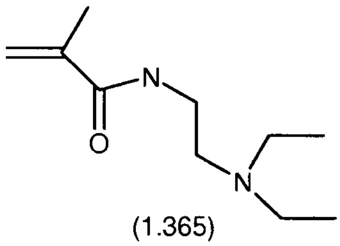

(1.365)

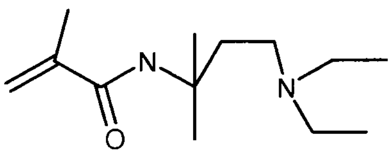

(1.969)

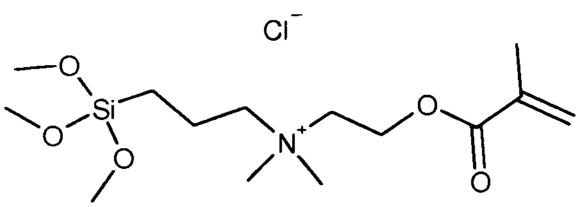

(logp calculé = -5)

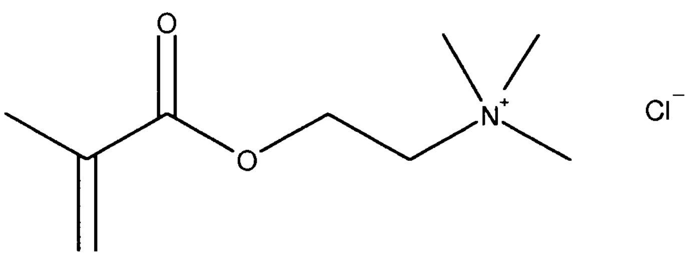

(logp calculé = -5)

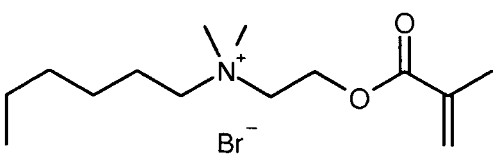

(logp calculé = -1)

Parmi les monomères de formule (II), on peut citer la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.

Parmi les monomères cationiques hydrophiles tout particulièrement préférés susceptibles d'être utilisés, on peut citer les monomères suivants :

- le (méth)acrylamide de diméthylaminopropyle, le (méth)acrylamide de diméthylaminoéthyle,
- le (méth)acrylate de diéthylaminoéthyle, le (méth)acrylate de diméthylaminoéthyle, le (méth)acrylate de morpholinoéthyle;
- la vinylimidazole, la vinylpyridine,
  et les monomères ci-après :

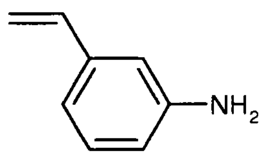

(1,418)

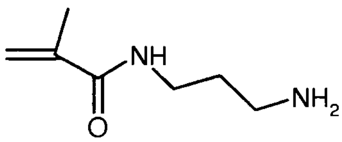

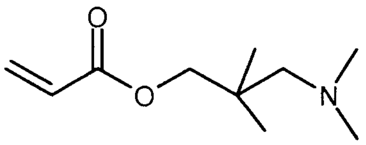

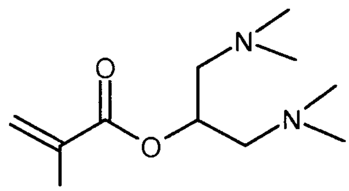

Les motifs amines peuvent être neutralisés, totalement ou partiellement, par des acides carboxyliques, possédant de préférence une chaîne alkyle linéaire ou ramifiée, ayant au moins 6 atomes de carbone, notamment de 6 à 32 atomes de carbone, voire de 12 à 24 atomes de carbone.

Les acides carboxyliques peuvent comporter, en outre, un ou plusieurs hétéroatomes choisis parmi O et N, par exemple sous la forme de groupes hydroxyles. On peut notamment citer l'acide acétique, l'acide alpha-hydroxyéthanoïque, l'acide alpha-hydroxyoctanoïque, l'acide alpha-hydroxycaprylique, l'acide ascorbique, l'acide benzoïque, l'acide béhénique, l'acide caprique, l'acide caproïque, l'acide caprylique, l'acide citrique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, et leurs mélanges.

La neutralisation peut être effectuée avant, pendant ou après la réaction de polymérisation. De préférence, la neutralisation est effectuée pendant la polymérisation.

Les motifs amines du polymère peuvent également être quaternisés, totalement ou partiellement. La quaternisation peut être effectuée avant ou après la réaction de polymérisation, à l'aide d'agents de quaternisation connus pour réaliser de telles réactions comme par exemple les dérivés halogénés (par exemple, halogénure d'alkyle, notamment en C6-C32, notamment bromure d'octyle).

Le polymère éthylénique selon l'invention peut comprendre un seul monomère cationique hydrophile ou un mélange de tels monomères cationiques hydrophiles.

Ainsi, dans un premier mode de réalisation de l'invention, les polymères présents dans la dispersion selon l'invention peuvent être issus uniquement de la polymérisation d'un ou plusieurs monomères cationiques hydrophiles,

qui vont donc représenter 100% en poids du poids total de monomères initiaux.

Toutefois, selon un second mode de réalisation de l'invention, les polymères présents dans la dispersion peuvent être issus de la polymérisation d'un ou plusieurs monomères cationiques hydrophiles, et d'un ou plusieurs monomères additionnels, qui seront donc de log p supérieur à 2, et/ou non cationiques, et qui peuvent donc être présents à raison de 0,01 à 50% en poids, notamment de 0,5 à 49% en poids, voire de 1 % à 48% en poids, et encore mieux de 2 à 45% en poids, préférentiellement de 5 à 40% en poids, et encore de 15 à 35% en poids par rapport au poids total de monomères.

Dans ce cas, les monomères cationiques hydrophiles représentent 50% à 99,99% en poids, voire de 51 % à 99,5% en poids, notamment de 52% à 99% en poids, et encore mieux de 55% à 98% en poids, préférentiellement de 60 à 95% en poids, et encore de 65 à 85% en poids, par rapport au poids total de monomères.

Les monomères additionnels peuvent notamment être choisis parmi les monomères suivants, seuls ou en mélange :

- (i) les esters de l'acide (méth)acrylique de formule $CH_2$=CHCOOR4 ou $CH_2$=C($CH_3$)COOR4 avec R4 représentant une chaîne carbonée, notamment hydrocarbonée (alkyle), ayant 1 à 32 atomes de carbone, linéaire, cyclique ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F).

En particulier, la chaîne carbonée R4 peut être :

- un groupe alkyle en C1-C32;
- un groupe cycloalkyle en C3 à C8;
- un groupe aryle en C6 à C20 ;
- un groupe aralkyle en C7 à C30 (groupe alkyle en C1 à C4);
- un groupe hétérocyclique de 4 à 12 chaînons contenant un ou plusieurs hétéroatomes choisis parmi O, N et S;
- un groupe hétérocycloalkyle (alkyl de C1 à C4) tel qu'un groupe furfuryle;

lesdits groupes alkyle, cycloalkyle, aryle, aralkyle, hétérocyclique ou hétérocycloalkyle pouvant comprendre intercalé (s) un ou plusieurs hétéroatomes choisis parmi O, N, S et/ou être substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, et/ou lesdits groupes alkyle pouvant, en outre, être éventuellement substitués par un ou plusieurs substituants choisis parmi le groupe hydroxyle et les atomes d'halogène (Cl, Br, I et F).

On peut ainsi citer les (méth)acrylates de méthyle, d'éthyle, de propyle, de n-butyle, d'isobutyle, de 2-éthylhexyle, d'octyle, d'isooctyle, d'isodécyle, de décyle, de lauryle, de tridécyle, de dodécyle, de myristyle, de cétyle, de palmityle, de stéaryle, de béhényle, d'oléyle; de tertiobutyle, de t-butylcyclohexyle, de t-butylbenzyle, de furfuryle et d'isobornyle; le (méth)acrylate de 2-hydroxyéthyle, le (méth)acrylate d'éthoxyéthyle; l'acrylate de 2-méthoxyéthyle et l'acrylate d'hydroxypropyle.

- (ii) les (méth)acrylamides de formule $CH_2$=CHCONR5R'5 ou $CH_2$=C($CH_3$)CONR5R'5 dans lesquelles R5 et R'5, identiques ou différents, représentent un atome d'hydrogène ou une chaîne carbonée, notamment hydrocarbonée (alkyle), ayant 6 à 28 atomes de carbone, linéaire, cyclique ou ramifiée, saturée ou insaturée, éventuellement aromatique, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F).

En particulier, la chaîne carbonée R5 et/ou R'5 peut être :

- un groupe alkyle en C6-C28;
- un groupe cycloalkyle en C6 à C8;
- un groupe aryle en C5 à C20 ;
- un groupe aralkyle en C5 à C28 (groupe alkyle en C1 à C4);
- un groupe hétérocyclique de 4 à 12 chaînons contenant un ou plusieurs hétéroatomes choisis parmi O, N et S;
- un groupe hétérocycloalkyle (alkyl de C1 à C4) tel qu'un groupe furfuryle;

lesdits groupes alkyle, cycloalkyle, aryle, aralkyle, hétérocyclique ou hétérocycloalkyle pouvant comprendre intercalé (s) un ou plusieurs hétéroatomes choisis parmi O, N, S et/ou être substitué par un ou plusieurs substituants choisis parmi le groupe hydroxyle, les atomes d'halogène et les groupes alkyles en C1-C4, linéaires ou ramifiés dans lesquels

se trouve(nt) éventuellement intercalé(s) un ou plusieurs hétéroatomes choisis parmi O, N, S et P, et/ou lesdits groupes alkyle pouvant, en outre, être éventuellement substitués par un ou plusieurs substituants choisis parmi le groupe hydroxyle et les atomes d'halogène (Cl, Br, I est F).

Des exemples de tels monomères sont la N-tertbutyl(méth)acrylamide, la N-butyl(méth)acrylamide, la N-isobutyl(méth)acrylamide, la N-propylméthacrylamide, la N-isopropylméthacrylamide, la N-hexyl(méth)acrylamide, la N-2-éthylhexyle(méth)acrylamide, la N-octyl(méth)acrylamide, la N-isooctyl(méth)acrylamide, la N-nonyl(méth)acrylamide, la N-undécyl(méth)acrylamide, la N-dodécyle(méth)acrylamide, la N-tridécyle(méth)acrylamide, la N-tétradécyle(méth) acrylamide, la N-hexadécyle(méth)acrylamide, la N-palmityle(méth)acrylamide, la N-octadécyle(méth)acrylamide, la N-docosanoyle(méth)acrylamide, la N-octadécénoyle(méth)acrylamide, la N-cyclohexyle(méth)acrylamide, la N-phényle (méth)acrylamide, la N-isobornyle(méth)acrylamide, la N-benzyle(méth)acrylamide, la N,N-dibutyl (méth)acrylamide.

- (iii) les esters vinyliques de formule $CH_2$=CH-OCO-R6 avec R6 représentant une chaîne carbonée, notamment hydrocarbonée, ayant 1 à 12 atomes de carbone, linéaire ou ramifiée, saturée ou insaturée, parmi lesquels on peut citer l'acétate de vinyle, le butyrate (ou butanoate) de vinyle, l'éthylhexanoate de vinyle, le néononanoate de vinyle, le néododécanoate de vinyle, le propionate de vinyle, l'hexanoate de vinyle, l'éthylhexanoate de vinyle, l'octanoate de vinyle, le décanoate de vinyle, le pivalate de vinyle, le palmitate de vinyle, le stéarate de vinyle, le cyclohexanoate de vinyle, le benzoate de vinyle, le 4-tert-butylbenzoate de vinyle;
- (iv) les éthers de vinyle de formule $CH_2$=CHOR7 avec R7 représentant une chaîne carbonée, notamment hydrocarbonée, ayant 1 à 12 atomes de carbone, linéaire ou ramifiée, saturée ou insaturée; parmi lesquels on peut citer l'éthylvinyléther, l'éthylhexylvinyléther, le butylvinyléther; l'isobutylvinyléther, le cyclohexylvinyléther, l'octylvinyléther, le décylvinyléther, le dodécylvinyléther, l'hexadécylvinyléther et l'octadécylvinyléther.
- (v) les composés vinyliques de formule $CH_2$=CHR8 dans laquelle R8 est
- un groupe hydroxyle;
- un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
- un groupe cycloalkyle en $C_3$ à $C_8$ tel que cyclohexane,
- un groupe aryle en $C_6$ à $C_{20}$ tel que phényle,
- un groupe aralkyle en $C_7$ à $C_{30}$ (groupe alkyle en $C_1$ à $C_4$) tel que 2-phényléthyle ou benzyle,
- un groupe hétérocyclique de 4 à 12 chaînons contenant un ou plusieurs hétéroatomes choisis parmi O, N, et S,
- un groupe hétérocycloalkyle (alkyle de 1 à 4 carbones), tel que furfuryle, furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I est F).

Des exemples de monomères vinyliques sont le vinylcyclohexane, le styrène, le vi-nylcaprolactame, le méthylstyrène; le 4-tert-butylstyrene, le 4-acétoxystyrene; le 4-méthoxystyrene, le 3-méthylstyrene; le 4-méthylstyrene, le 2-chlorostyrene, le 3-chlorostyrene, le 4-chlorostyrene; le 2,6-dichlorostyrene; le 2,4-dimethylstyrene; le 2,5-dimethylstyrene; le 3,5-éthoxystyrene; le 2,4-éthoxystyrene, le vinylbutyral; le chlorure de vinyle; le vinylformal; le vinylidène chlorure, le vinylidène fluorure, le 2-vinylnaphtalène; le N-méthyl maléimide; le 1-octene, le 1-butene; le chlorobutadiene, le chlorotrifluoroethylene; le cis-isoprene, le trans-isoprene; le 1-octadecene; le butadiène; l'hexadécène, l'eicosène, le 4-fluorostyrene.

- (vi) des monomères multifonctionnels, notamment difonctionnels, tels que les di(méth)acrylates ou tri(méth)acrylates, et en particulier choisis parmi le 1,4-butanedioldiméthacrylate, le 1-6 hexanedioldimethacrylate, le 1,12-dodecanedioldiméthacrylate, le diallylphtalate, le divinylbenzene, le poly(éthylène glycol) diméthacrylate, et leurs mélanges.

- (vii) l'acide (méth)acrylique; l'anhydride maléique; l'acide crotonique, l'acide itaco-nique, l'acide fumarique, l'acide maléique, l'acide diacrylique, l'acide diméthylfuma-rique, l'acide citraconique, l'acide acrylamidopropanesulfonique, l'acide 2-acrylamido2-méthylpropanesulfonique, l'acide styrènesulfonique, l'acide vinylben-zoïque, l'acide vinylphosphorique, l'acide vinylsulfonique, l'acide vinylbenzène sulfonique, l'acide acrylamidoglycolique de formule CH2=CH-CONHCH(OH)COOH, l'acide vinylphosphonique; le (méth)acrylate de sulfopropyle, le (méth)acrylate de sulfoéthyle, la vinylméthylsulfone, le 2-(méthacryloyloxy)éthylphosphate de formule $CH_2=C(CH_3)COOC_2H_4OP(O)(OH)_2$; ainsi que leurs sels; et leurs mélanges.

- (viii) les (méth)acrylates de poly(éthylène glycol), les (méth)acrylates d'alkylpoly(éthylène glycol), et plus particulièrement les méthacrylates de méthylpoly(éthylène glycol),

De préférence, les monomères additionnels peuvent être choisis parmi les esters de l'acide (méth)acrylique en C1-C18, les (méth)acrylamides, et notamment parmi les (méth)acrylates et (méth)acrylamides de méthyle, d'éthyle, de propyle, de n-butyle, d'isobutyle, de 2-éthylhexyle, de tertiobutyle, d'isooctyle, de décyle, de myristyle, de stéaryle; ou le styrène, l'éthylhexylvinylether, le dodécylvinyléther, l'hexanoate de vinyle; l'acrylate de 2-hydroxyéthyle et le méthacrylate de poly(éthylene glycol), ainsi que leurs mélanges.

De préférence, le polymère selon l'invention présente une présente une température de transition vitreuse (Tg) inférieure ou égale à 30 °C, en particulier comprise entre - 150°C et 25°C, notamment entre -70°C et 20°C, préférentiellement entre -50°C et 0°C.

Pour ce faire, les polymères présents dans la dispersion selon l'invention peuvent être issus de la polymérisation d'un ou plusieurs monomères de Tg inférieure ou égale à 30°C (dits de basse Tg), de préférence comprise entre -150°C et 25°C, notamment entre -70°C et 20°C, préférentiellement entre -50°C et 0°C, qui peuvent représenter 100% en poids du poids total de monomères initiaux, ou bien qui peuvent être en mélange avec un ou plusieurs monomères de Tg supérieure à 30°C (dits de haute Tg), mais présents en une quantité telle que la Tg globale du polymère est inférieure ou égale à 30°C. Dans ce second cas, le monomère de haute Tg seul ou en mélange, peut être présent à raison de 0,01 à 50% en poids, par rapport au poids total des monomères, notamment de 0,1 à 40% en poids, voire de 1 à 30% en poids, ou encore de 5 à 15% en poids; le monomère de basse Tg, seul ou en mélange, étant alors présent à raison de 50 à 99,99% en poids, notamment de 60 à 99,9% en poids, voire de 70 à 99% en poids, ou encore de 85 à 95% en poids, par rapport au poids total de monomères.

Dans la présente invention, les Tg (ou température de transition vitreuse) indiquées sont des Tg théoriques déterminées à partir des Tg théoriques des monomères constitutifs du polymère, que l'on peut trouver dans un manuel de référence, tel que le Polymer Handbook, 4th éd. (Brandrup, Immergut, Grulke), 1999, John Wiley, selon la relation suivante, dite Loi de Fox :

$$\frac{1}{Tg} = \sum_i \left(\frac{\varpi i}{Tgi}\right)$$

wi étant la fraction massique du monomère i dans le polymère et Tgi étant la température de transition vitreuse de l'homopolymère du monomère i (exprimée en Kelvin).

Dans la présente description, on désigne par "monomère de Tg", le monomère dont l'homopolymère a une telle température de transition vitreuse.

L'homme du métier saura, sur la base de la loi de Fox et de ses connaissances générales, déterminer les quantités de chaque monomère susceptible d'être présent dans le polymère de la dispersion, de manière à toujours obtenir au final une dispersion de polymère ayant une Tg dans la gamme souhaitée.

De préférence, le polymère ne présente qu'une température de transition vitreuse. Toutefois, il peut présenter plusieurs températures de transition vitreuse, notamment deux Tg; dans ce cas, de préférence, la Tg la plus basse est inférieure à 30°C. Les monomères de basse Tg peuvent être choisis parmi les monomères cationiques hydrophiles et/ou les monomères additionnels; il en est de même des monomères de haute Tg.

Les polymères utilisables dans le cadre de la présente invention ont de préférence un poids moléculaire moyen en nombre (Mn) compris entre 2000 à 1 000 0000, notamment entre 3000 et 800 000, et encore mieux entre 4000 et 500 000, voire entre 10 000 et 300 000.

La dispersion de particules de polymères selon l'invention comprend donc un milieu non aqueux, dans lequel sont dispersées lesdites particules.

Ce milieu non aqueux est constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés.

Le paramètre de solubilité global δ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = (d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés liquides non aqueux ayant un paramètre de solubilité global selon l'espace de solubilité de HANSEN inférieur ou égal à 20 $(MPa)^{1/2}$, on peut citer les corps gras liquides, notamment les huiles, qui peuvent être choisies parmi les huiles naturelles ou synthétiques, carbonées, hydrocarbonées, fluorées et/ou siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.

On peut également citer les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

On peut également citer les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.

Parmi les composés non aqueux susceptibles d'être employés, on peut également citer les monoalcools ayant un paramètre de solubilité global selon l'espace de solubilité de HANSEN inférieur ou égal à 20 $(MPa)^{1/2}$, c'est-à-dire les monoalcools gras aliphatiques ayant au moins 6 atomes de carbone, notamment 6 à 32, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

De préférence, le milieux non aqueux comprend des huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Le choix du milieu non aqueux peut être effectué aisément par l'homme du métier en fonction de la nature des monomères constituant le polymère et/ou de la nature du stabilisant.

La dispersion de polymère peut être fabriquée comme décrit dans le document EP-A-749747. D'une manière générale, la polymérisation peut être effectuée en dispersion, c'est-à-dire par précipitation du polymère au cours de formation, avec protection des particules formées à l'aide d'un stabilisant.

On peut donc préparer un mélange comprenant les monomères initiaux ainsi qu'un amorceur radicalaire. Ce mélange est dissous dans un solvant de synthèse.

Les monomères sont solubles dans le milieu réactionnel, alors que le polymère n'y est pas soluble. Au fur et à mesure de la polymérisation, le polymère va précipiter et se trouver stabilisé par le stabilisant présent. On obtient ainsi des particules de polymères protégées en surface par le stabilisant.

On peut directement effectuer la polymérisation dans le milieu non aqueux qui peut donc jouer également le rôle de solvant de synthèse.

On peut également effectuer la polymérisation dans un solvant de synthèse, puis effectuer ensuite un échange de solvant, en remplaçant le solvant de synthèse par le milieu non aqueux.

Ainsi, lorsque le milieu non aqueux choisi est une huile non volatile, hydrocarbonée ou siliconée, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc de préférence un solvant de synthèse tel que les monomères initiaux, et l'amorceur radicalaire, y sont solubles, et les particules de polymère obtenu y sont insolubles afin qu'elles y précipitent lors de leur formation.

En particulier, on peut choisir le solvant de synthèse parmi les alcanes tels que l'heptane, l'isododécane ou le cyclohexane.

Lorsque le milieu non aqueux choisi est une huile volatile, hydrocarbonée ou siliconée, on peut directement effectuer la polymérisation dans ladite huile qui joue donc également le rôle de solvant de synthèse. Les monomères doivent de préférence également y être solubles, ainsi que l'amorceur radicalaire, et le polymère obtenu doit y est insoluble.

Les monomères sont de préférence présents dans le solvant de synthèse, avant polymérisation, à raison de 5-80% en poids. La totalité des monomères peut être présente dans le solvant avant le début de la réaction, ou une partie des monomères peut être ajoutée au fur et à mesure de l'évolution de la réaction de polymérisation. L'amorceur radicalaire peut être, par exemple, un composé azoïque ou peroxyde tels que l'azo-bis-isobutyronitrile ou le tertiobutylperoxy-2-éthyl hexanoate.

Les particules de polymère sont stabilisées en surface.

Dans un premier mode de réalisation, les particules peuvent être stabilisées en surface au fur et à mesure de la polymérisation, grâce à un stabilisant qui peut être notamment un polymère séquencé, un polymère greffé, et/ou un polymère statistique, seul ou en mélange. La stabilisation peut être effectuée par tout moyen connu, et en particulier par polymérisation en présence du stabilisant.

De préférence, le stabilisant est présent dans le mélange au départ de la polymérisation. Toutefois, il est également possible de l'ajouter en continu, notamment lorsqu'on ajoute également des monomères en continu.

Dans un second mode de réalisation, le polymère peut être synthétisé dans un solvant de synthèse, puis mis en dispersion dans un milieu non aqueux de dispersion par ajout du dispersant, et le solvant de synthèse évaporé.

Par dispersant, on entend un polymère non lié par liaison covalente au polymère éthylénique défini ci-dessus.

On peut utiliser 0,1 à 30% en poids de stabilisant par rapport au poids du mélange initial de monomères, et de préférence de 1 à 20% en poids, préférentiellement de 2 à 15% en poids, voire de 3 à 10% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant comportant une partie (séquences, greffons ou autre), présentant une certaine affinité pour le polymère formé lors de la polymérisation.

Lorsqu'on utilise un polymère statistique en tant que stabilisant, on le choisit de manière à ce qu'il possède une quantité suffisante de groupements le rendant soluble dans le solvant de synthèse envisagé.

Parmi les polymères greffés, on peut citer les polymères siliconés greffés avec une chaîne hydrocarbonée et les polymères hydrocarbonés greffés avec une chaîne siliconée.

Conviennent également les copolymères greffés ayant par exemple un squelette insoluble de type polyacrylique avec des greffons solubles de type acide poly(12-hydroxy stéarique).

On peut également citer, comme polymère stabilisant :

- (a) les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées;

Comme copolymères blocs greffés ou séquences comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

Lorsque les copolymères blocs greffés ou séquencés comprennent au moins un bloc de type polyorganosiloxane et au moins un bloc polyéther, le bloc polyorganopolysiloxane peut être notamment un polydiméthylsiloxane ou bien encore un polyalkyl($C_2$-$C_{18}$)méthylsiloxane; le bloc polyéther peut être un polyalkylène en $C_2$-$C_{18}$, en particulier polyoxyéthylène et/ou polyoxypropylène. On peut ainsi utiliser les diméthicones copolyol ou encore les alkyl($C_2$-$C_{18}$) méthicones copolyol, éventuellement réticulés. On peut par exemple utiliser les diméthicones copolyols (silicone polyéther) vendus sous la dénomination "DC3225C" ou "DC5225C" par la société DOW CORNING, ou le lauryl méthicone copolyol vendu sous la dénomination "DC Q2-5200 par la société "DOW CORNING".

On peut aussi citer le lauryl diméthicone copolyol crosspolymer, par exemple le KSG31 ou KSG32 de Shin-Etsu, le cétyl diméthicone copolyol tel que le DMC 3071 de GE, et le diméthicone copolyol PPG-3-oléyl éther tel que le KF-6026 de Shin Etsu, ainsi que le diméthicone copolyol PEG-10 dimethicone, vendu par Shin Etsu sous la dénomination KF-6017.

- (b) les copolymères blocs, greffés ou séquencés, de (méth)acrylates d'alkyle en C1-C4 et de (méth)acrylates d'alkyle en C8-C30. On peut citer le copolymère méthacrylate de stéaryle/méthacrylate de méthyle.

- (c) les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.

Notamment, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquences, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthacrylate de méthyle)/polyisobutylène ou les copolymères greffés à squelette poly(méthacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également utiliser comme stabilisant, des composés tels que :

- (d) les alkyl-dimethicones dans lesquels le groupement alkyl comprend 6 à 32 atomes de carbone, tels que le lauryle méthicone et le stéaryle méthicone, notamment Si tec LDM 3107 d'ISP, le cetyl dimethicone tel que l'ABIL Wax 9801, le behenoxydimethicone tel que l'ABIL 5440 de Goldschmidt

- (e) les dimethiconol ester de formule :

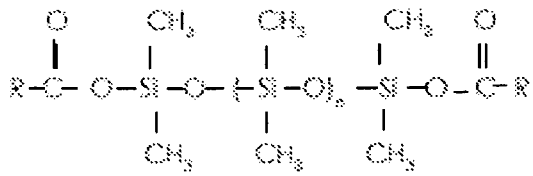

dans laquelle R est un radical alkyle ayant 6 à 32 atomes de carbone, tel que le dimethiconol behenate, et notamment les produits ULTRABEE de NOVEON et Pecosil DB de Phoenix Chemical.

- (f) les alkylamidoamines ayant notamment 6 à 60 atomes de carbone, notamment 12 à 50, telles que la behenamidopropyldimethylamine et notamment le Catemol 220 de Phoenix Chemical, de formule :

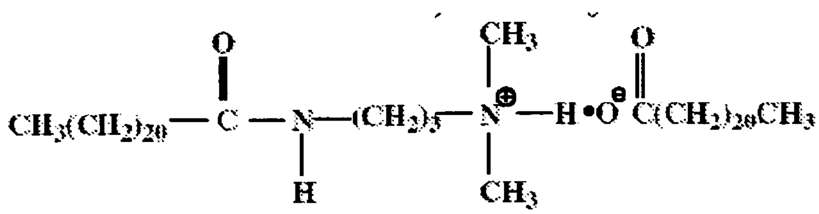

- (g) les copolymères comprenant au moins une partie polyorganosiloxane et des groupements fluorés, et notamment les silicones fluorées ou fluorosilicones qui peuvent être représentées par la formule :

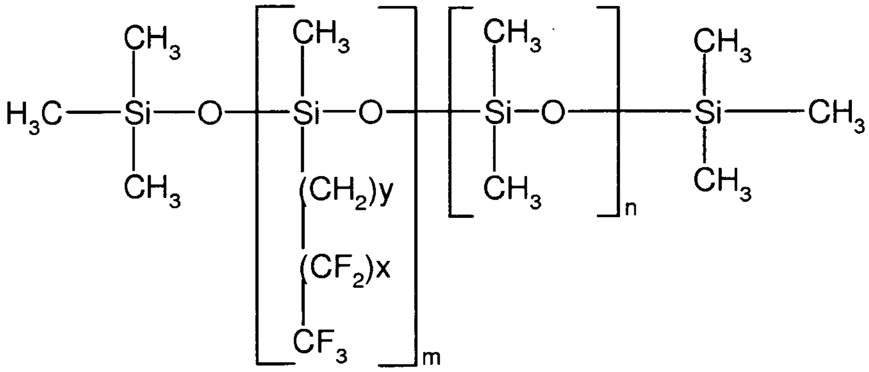

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000; et plus particulièrement les perfluorononyldimethicone, tels que ceux vendus sous la dénomination PECOSIL FSH-150 et 300 ou PECOSIL FSL-150 et 300 par Phoenix Chemical;

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

De manière particulièrement préférée, la dispersion selon l'invention est telle que :

1/ les monomères cationiques hydrophiles sont choisis parmi :

- le (méth)acrylamide de diméthylaminopropyle, le (méth)acrylamide de diméthylaminoéthyle,
- le (méth)acrylate de diéthylaminoéthyle, le (méth)acrylate de diméthylaminoéthyle, le (méth)acrylate de morpholinoéthyle;
- la vinylimidazole, la vinylpyridine,
  et les monomères ci-après :

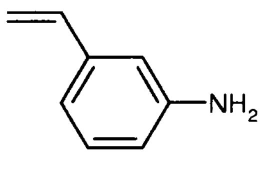

(1,418)

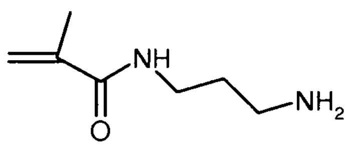

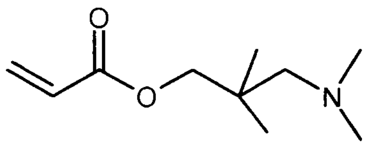

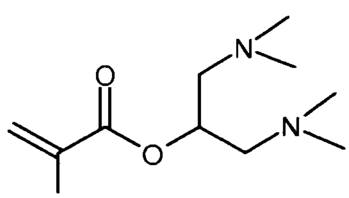

et/ou

2/ l'agent stabilisant est choisi parmi :

- les silicones fluorées ou fluorosilicones de formule :

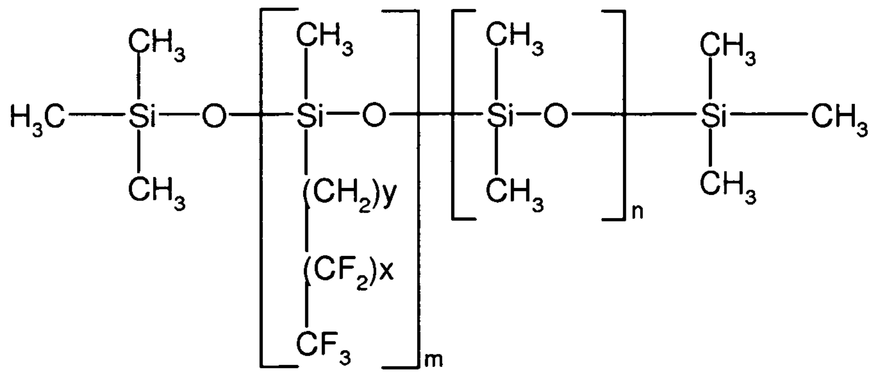

dans laquelle x=8, y = 2 ou 3, et m et n sont tels que le poids moléculaire du composé est compris entre 5000 et 15000; et
- les copolymères blocs greffés ou séquencés comprenant au moins un bloc de type polyorganosiloxane et au moins un bloc d'un polymère issu d'une polycondensation, notamment de type polyéther, polyester ou polyamide; en particulier, un bloc polyéther de type polyalkylène en $C_2$-$C_{18}$, notamment polyoxyéthylène et/ou polyoxypropylène.

et/ou

3/ le composé liquide non aqueux est choisi parmi les huiles siliconées volatiles, notamment cycliques ou linéaires, telles que les cyclodiméthylsiloxanes et les diméthylsiloxanes linéaires, et/ou les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Il est possible d'ajouter à la dispersion de polymères, un plastifiant de manière à abaisser la Tg des polymères utilisés. Le plastifiant peut être choisi parmi les plastifiants usuellement utilisés dans le domaine d'application et notamment parmi les composés susceptibles d'être des solvants du polymère. Le plastifiant peut être intégré lors de la synthèse ou ajouté une fois la synthèse réalisée.

Les dispersion obtenues selon l'invention peuvent alors être utilisées dans une composition notamment cosmétique ou pharmaceutique, qui comprend par ailleurs un milieu cosmétiquement ou pharmaceutiquement acceptable.

Selon l'application envisagée, on peut utiliser des dispersion de polymères filmifiables ou non filmifiables, dans un milieu non aqueux comprenant des huiles volatiles ou non volatiles.

La composition peut alors comprendre, selon l'application envisagée, les constituants habituels à ce type de composition.

Parmi ces constituants, on peut citer les cires, les huiles, les gommes et/ou les corps gras pâteux, d'origine végétale, animale, minérale ou de synthèse, voire siliconés, et leurs mélanges.

Parmi les cires susceptibles d'être présentes dans la composition selon l'invention, on peut citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La composition selon l'invention peut également comprendre des huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée. Parmi les huiles susceptibles d'être présentes dans la composition selon l'invention, on peut citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide

lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

La composition selon l'invention peut également comprendre une ou plusieurs matières colorantes choisies parmi les composés pulvérulents et/ou les colorants liposolubles ou hydrosolubles, par exemple à raison de 0,01 à 70% du poids total de la composition.

Les composés pulvérulents peuvent être choisis parmi les pigments et/ou les nacres et/ou les charges habituellement utilisés dans les compositions cosmétiques ou pharmaceutiques. Avantageusement, les composés pulvérulents représentent 0,1 à 50% du poids total de la composition et mieux de 1 à 40%.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyllysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0 à 20 % et notamment 0,01 à 20 % du poids de la compositions et mieux de 0,1 à 6 %. Les colorants hydrosolubles sont par exemple le jus de betterave, le bleu de méthylène et peuvent représenter jusqu'à 6 % du poids total de la composition.

La composition peut également comprendre des tensioactifs, qui peuvent être choisis parmi les tensioactifs cationiques, anioniques, amphotères et non ioniques, et leurs mélanges. Ces tensioactifs peuvent être présents à raison de 0,01 à 30% en poids, notamment 0,05 à 20% en poids, par rapport au poids total de la composition.

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des céramides, des filtres solaires, des polymères, des épaississants, des gélifiants. Bien entendu l'homme du métier veillera à choisir ce ou ces additifs et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.

Elle trouve notamment une application particulièrement intéressante dans le domaine capillaire, notamment

pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques, des mousses ou des sprays. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux. Les compositions selon l'invention peuvent être des compositions détergentes telles que des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux. Les compositions peuvent encore se présenter sous forme de produit de soin du cheveu (masque capillaire), de laque, gel ou mousse coiffant.

L'invention a encore pour objet un procédé de traitement cosmétique des matières kératiniques, notamment de la peau du corps ou du visage, des ongles, des cheveux et/ou des cils, comprenant l'application sur lesdites matières d'une composition cosmétique telle que définie précédemment.

Ce procédé selon l'invention permet notamment le maintien de la coiffure, le traitement, le soin ou le lavage ou le démaquillage de la peau, des cheveux ou de toute autre matière kératinique.

L'invention est illustrée plus en détail dans les exemples suivants, dans lesquels la Tg des polymères est calculée selon la loi de Fox.

**<u>Exemple 1</u>**

Dans un réacteur de 500 ml muni d'un réfrigérant, d'une agitation et d'une ampoule d'addition de 250 ml, on introduit les constituants du pied de cuve. On chauffe à 80°C en 1 heure; puis on ajoute les constituants de la coulée, en 1 heure. On maintient la température à 80°C pendant 5 heures.

| | Pied de cuve (Q) | Coulée (g) | Quantité totale(g) |
|---|---|---|---|
| Méthacrylate de diméthylaminoéthyle (lop p = 1,5) | 10 | 30 | 40 |
| Neutralisant (acide palmitique) | 17 | 49 | 66 |
| Stabilisant : PEG-10 dimethicone (KF-6017 de Shin Etsu) | 2,5 | 2,5 | 5 |
| Myristate d'isopropyle | 200 ml | 100 ml | 300 ml |
| Amorceur (Triqonox 21 S) | 0,3 | 0,3 | 0,6 |

On obtient une dispersion orangée de polymère dans le myristate d'isopropyle, ayant une teneur en matière sèche de 26%. Le monomère cationique hydrophile représente 100% en poids du poids total de monomères.

**<u>Exemple 2</u>**

Dans un réacteur de 500 ml muni d'un réfrigérant, d'une agitation et d'une ampoule d'addition de 250 ml, on introduit les constituants du pied de cuve. On chauffe à 80°C en 1 heure; puis on ajoute les constituants de la coulée, en 1 heure. On maintient la température à 80°C pendant 5 heures.

| | Pied de cuve (g) | Coulée (g) | Quantité totale (g) |
|---|---|---|---|
| Méthacrylate de diméthylaminoéthyle (log p = 1,5) | 5 | 15 | 20 |
| Acrylate de 2-éthyle hexyle (log p = 4,33) | 5 | 10 | 15 |
| Neutralisant (acide palmitique) | 8,5 | 24,5 | 33 |

(suite)

| | Pied de cuve (g) | Coulée (g) | Quantité totale (g) |
|---|---|---|---|
| Stabilisant * | 2,5 | 2,5 | 5 |
| Myristate d'isopropyle | 200 ml | 150 ml | 350 ml |
| Amorceur (Trigonox 21 S) | 0,5 | 0,5 | 1 |
| * Stabilisant : Cyclopentasiloxane (and) PEG/PPG-18/18 Dimethicone (DC5225C de Dow Corning) | | | |

On obtient une dispersion orangée de polymère dans le myristate d'isopropyle, ayant une teneur en matière sèche de 20%. Le monomère cationique hydrophile représente 57% en poids du poids total de monomères.

**Exemple 3**

On prépare les compositions suivantes :

| | Composition A | Composition B |
|---|---|---|
| Dispersion de l'exemple 1 | 1,0 % MA de polymère | - |
| Dispersion de l'exemple 2 | - | 1,0 % MA de polymère |
| Dimethicone copolyol/D5 (DC5225C) | 0,5% MA diméthicone copolyol | 0,5% MA diméthicone copolyol |
| Cyclopentasiloxane (D5) | 10,0 % | 10,0 % |
| Chlorure de triméthylbéhénylammonium | 1,2% | 1,2% |
| Propylèneglycol | 2,5% | 2,5% |
| Conservateur, parfum | qs | qs |
| Acide citrique/soude | qs pH 6,5 | qs pH 6,5 |
| Eau | qsp 100% | qsp 100% |

On applique 2 grammes de chaque composition A et B sur des mèches de cheveux sensibilisés de 20 cm de longueur et pesant 2,7 grammes. Les mèches sont malaxées, laissées à poser 5 minutes puis rincées. On évalue la tonicité de la boucle, le toucher des cheveux ainsi que le démêlage des mèches au peigne. Le cheveu témoin n'a subi aucun traitement

On note que les 2 compositions selon l'invention A et B apportent un niveau de tonicité à la boucle quasi-identique; ce niveau est bien supérieur à celui obtenu avec la mèche témoin qui n'a subi aucun traitement.

**Exemple 4**

Dans un réacteur de 500 ml muni d'un réfrigérant, d'une agitation et d'une ampoule d'addition de 250 ml, on introduit les constituants du pied de cuve. On chauffe à 80°C en 1 heure, puis on ajoute les constituants de la coulée, en 1 heure. On maintient la température à 80°C pendant 5 heures.

| | Pied de cuve (g) | Coulée (g) | Quantité totale (g) |
|---|---|---|---|
| Méthacrylate de diméthylaminoéthyle | -- | 50 | 50 |
| Neutralisant (acide éthylcaproïque) | -- | 50 | 50 |
| Stabilisant * | 2,5 | 2,5 | 5 |
| Myristate d'isopropyle | 100 ml | 100 ml | 200 ml |

(suite)

| | Pied de cuve (g) | Coulée (g) | Quantité totale (g) |
|---|---|---|---|
| Amorceur (Trigonox 21S) | - | 1 | 1 |
| *PECOSIL FSH-150 de Phoenix Chemical | | | |

On obtient une dispersion de polymère dans le myristate d'isopropyle, ayant une teneur en matière sèche de 32%. Le monomère cationique hydrophile représente 100% en poids du poids total de monomères.

**Exemple 5**

Dans un réacteur de 500 ml muni d'un réfrigérant, d'une agitation et d'une ampoule d'addition de 250 ml, on introduit les constituants du pied de cuve. On chauffe à 80°C en 1 heure, puis on ajoute les constituants de la coulée, en 1 heure. On maintient la température à 80°C pendant 5 heures.

| | Pied de cuve (g) | Coulée (g) | Quantité totale (g) |
|---|---|---|---|
| Méthacrylate de diméthylaminoéthyle | 7,5 | 35 | 42,5 |
| Neutralisant (acide éthylcaproïque) | 7,5 | 35 | 42,5 |
| Acrylate de 2-éthylhexyle | 7,5 | 7,5 | 15 |
| Stabilisant * | 2,5 | 2,5 | 5 |
| Myristate d'isopropyle | 100 ml | 100 ml | 200 ml |
| Amorceur (Trigonox 21 S) | -- | 1 | 1 |
| *PECOSIL FSH-150 de Phoenix Chemical | | | |

On obtient une dispersion de polymère dans le myristate d'isopropyle, ayant une teneur en matière sèche de 33%. Le monomère cationique hydrophile représente 74% en poids du poids total de monomères.

**Exemple 6**

On prépare les compositions suivantes (en %):

| | Composition C | Composition D | Composition E Comparatif |
|---|---|---|---|
| Dispersion de l'exemple 4 | 1 % MA de polymère | - | - |
| Dispersion de l'exemple 5 | - | 1 % MA de polymère | - |
| Cetylalcool | 3% MA | 3% MA | 3% MA |
| Cetyl esters (mélange de myristate, palmitate, stéarate de myristyle, cétyle et stéaryle) | 0,25% MA | 0,25% MA | 0,25% MA |
| Alcool myristique | 0,75% MA | 0,75% MA | 0,75% MA |
| Huile de palme | 0,15% MA | 0,15% MA | 0,15% MA |
| Hydroxyéthylcellulose (PM= 1300.000) | 0,2% MA | 0,2% MA | 0,2% MA |
| Chlorure de cétyltriméthylammonium | - | - | 0,45% MA |
| Stéarylamidopropyldiméthylamine | - | - | 0,75% MA |
| Conservateur | Qs | Qs | Qs |

(suite)

| | | | |
|---|---|---|---|
| Eau | Qsp 100% | Qsp 100% | Qsp 100% |

On applique 2 grammes de chaque composition C, D et E sur des mèches de cheveux sensibilisés, de 20 cm de longueur et pesant 2,7 grammes. Les mèches sont malaxées, laissées à poser 5 minutes puis rincées. On évalue la tonicité de la boucle, le toucher des cheveux ainsi que le démêlage des mèches au peigne.

On note que les 2 compositions selon l'invention C et D apportent un niveau de tonicité à la boucle quasi-identique; ce niveau est bien supérieur à celui obtenu avec la mèche référence traitée avec la composition comparative E.

**Revendications**

**1.** Dispersion de particules d'au moins un polymère éthylénique, stabilisées en surface par un agent stabilisant, dans un milieu non aqueux constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés; **caractérisée par le fait que** ledit polymère éthylénique comprend de 50 à 100% en poids de monomère hydrophile cationique ayant un log p inférieur ou égal à 2, ou d'un mélange de tels monomères, par rapport au poids total de monomères.

**2.** Dispersion selon la revendication 1, dans laquelle le polymère comprend de 50 à 100% en poids, de monomère cationique hydrophile, seul ou en mélange, par rapport au poids total de monomères initiaux.

**3.** Dispersion selon l'une des revendications précédentes, dans laquelle le monomère hydrophile cationique présente une valeur de log p comprise entre -10 et 2.

**4.** Dispersion selon l'une des revendications précédentes, dans laquelle le monomère cationique hydrophile est choisi parmi, seul ou en mélange, les monomères de formules (I) et (II) suivantes, ainsi que leurs sels:

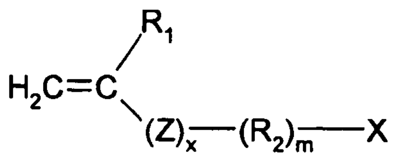

(I)

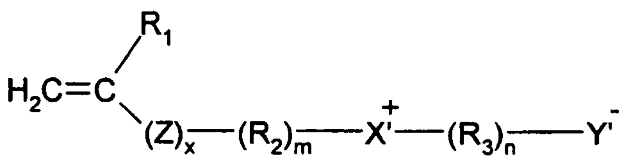

(II)

dans lesquelles :

- R1 est un atome d'hydrogène ou un radical hydrocarboné, linéaire ou ramifié, de type $C_pH_{2p+1}$, avec p étant un entier compris entre 1 et 12, inclus;
- Z est un groupement divalent choisi parmi -COO-, -CONH-, $-CONCH_3-$, -OCO-, - O-, $-SO_2-$ -CO-O-CO- ou $-CO-CH_2-CO-$;
- x est 0 ou 1,
- R2 et R3 sont, indépendamment l'un de l'autre, un radical divalent carboné, saturé ou insaturé, ou aromatique, linéaire, ramifié ou cyclique, de 1 à 30 atomes de carbone, comprenant ou non 1 à 18 hétéroatomes choisis parmi O, N, S, F, Si et P;
- m est 0 ou 1;
- n est compris entre 1 et 100;
- X est :

(a) un groupe guanidino ou un groupe amidino; ou

(b) un groupe de formule $-N(R_6)(R_7)$ avec R6 et R7 représentant, indépendamment l'un de l'autre, (i) un atome d'hydrogène, (ii) un groupement alkyle linéaire, ramifié ou cyclique, saturé ou insaturé, ou aromatique, comprenant de 1 à 18 atomes de carbone, comprenant ou non 1 à 10 hétéroatomes choisis parmi O, N, S, F, Si et P;

ou bien (iii) R6 et R7 forment avec l'atome d'azote un cycle de formule:

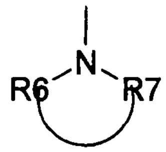

saturé ou insaturé, ou aromatique, comprenant au total 5 à 8 atomes; ledit premier cycle étant ou non fusionné avec un ou plusieurs autres cycles, saturés ou insaturés, ou aromatiques, comprenant chacun 5 à 7 atomes;

(c) un cycle :

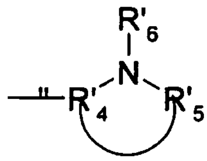

dans lequel R'4 et R'5 forment avec l'atome d'azote un cycle, saturé ou insaturé, ou aromatique, comprenant au total 5 à 8 atomes; ledit cycle étant ou non fusionné avec un ou plusieurs autres cycles, saturés ou insaturés, ou aromatiques, comprenant chacun 5 à 7 atomes; et R'6 est choisi parmi H, $-CH_3$ et $-C_2H_5$.

- X'+ est un groupe divalent de formule $-N^+(R_6)(R_7)-$ avec R6 et R7 représentant, indépendamment l'un de l'autre, (i) un atome d'hydrogène, (ii) un groupement alkyle linéaire, ramifié ou cyclique, ou aromatique, comprenant de 1 à 25 atomes de carbone, comprenant ou non 1 à 20 hétéroatomes choisis parmi O, N, S et P; ou (iii) R6 et R7 forment avec l'atome d'azote un cycle de formule :

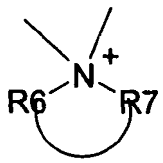

saturé ou insaturé, ou aromatique, comprenant au total 5 à 8 atomes; ledit premier cycle étant ou non fusionné avec un ou plusieurs autres cycles, saturés ou insaturés, ou aromatiques, comprenant chacun 5 à 7 atomes;

ou bien X'+ est un cycle divalent de formule:

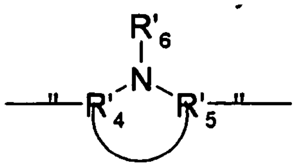

dans lequel R'4 et R'5 forment avec l'atome d'azote un cycle, saturé ou insaturé, ou aromatique, comprenant au total 5 à 8 atomes; ledit cycle étant ou non fusionné avec un ou plusieurs autres cycles, saturés ou insaturés, ou aromatiques, comprenant chacun 5 à 7 atomes; et R'6 est choisi parmi H, $-CH_3$ et $-C_2H_5$;

- Y'- est un groupement choisi parmi $-COO^{7}$, $-SO_3^{-'}$ $-OSO_3^-$, $-PO_3^{2-}$ et $-OPO_3^{2-}$.

**5.** Dispersion selon la revendication 4, dans laquelle X est choisi parmi :

- un groupe de formule $-N(R_6)(R_7)$ avec R6 et R7 représentant, indépendamment l'un de l'autre, (i) un atome d'hydrogène, (ii) un groupement alkyle linéaire, ramifié ou cyclique, saturé ou insaturé, ou aromatique, comprenant de 1 à 18 atomes de carbone, ou (iii) R6 et R7 forment avec l'atome d'azote un cycle de formule :

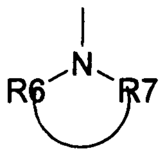

saturé ou insaturé, ou aromatique, comprenant au total 5 à 7 atomes;
- un cycle :

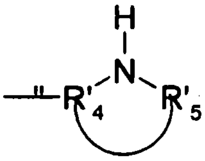

dans lequel R'4 et R'5 forment avec l'atome d'azote un cycle, saturé ou insaturé, ou aromatique, comprenant 5 à 7 atomes.

**6.** Dispersion selon l'une des revendications 4 à 5, dans laquelle X est choisi parmi un groupe $NH_2$, $N(CH_3)_2$, pyridinyle, imidazolyle, piperidinyl, piperazinyl ou morpholine.

**7.** Dispersion selon l'une des revendications précédentes, dans laquelle le monomère cationique hydrophile est choisi parmi, seul ou en mélange :

- le (méth)acrylamide de diméthylaminopropyle, le (méth)acrylamide de diméthylaminoéthyle, le (méth)acrylamide de diéthylaminopropyle;
- le (méth)acrylate de diéthylaminoéthyle, le (méth)acrylate de diméthylaminoéthyle, le (méth)acrylate de morpholinoéthyle; le (méth)acrylate de tert-butylaminoéthyle;
- la vinylimidazole, la vinylpyridine, la vinylamine,

et les monomères ci-après :

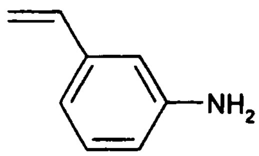

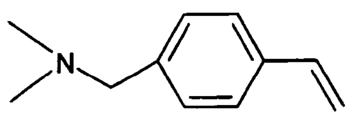

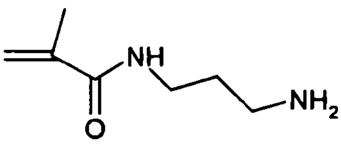

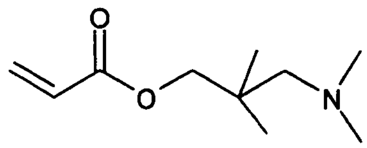

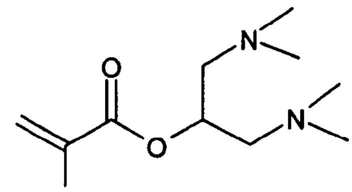

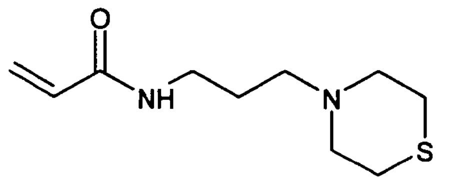

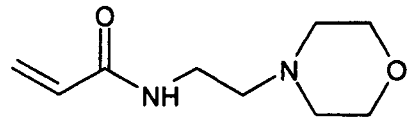

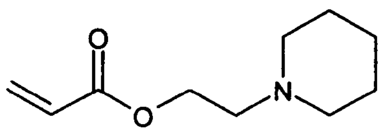

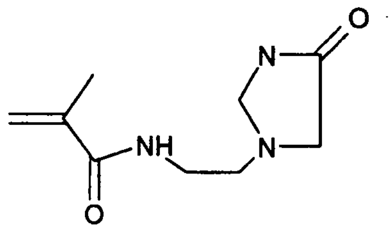

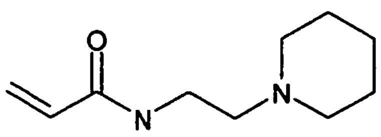

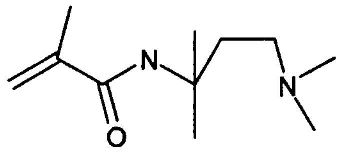

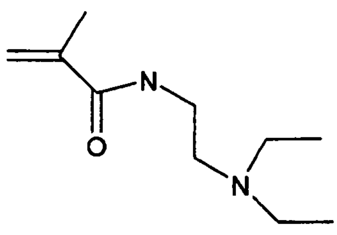

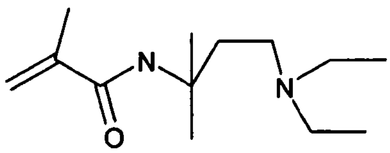

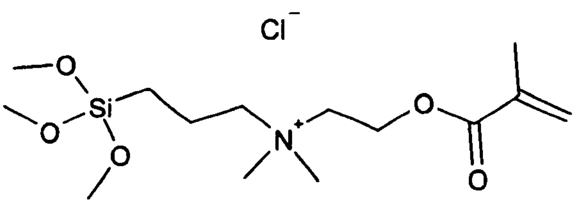

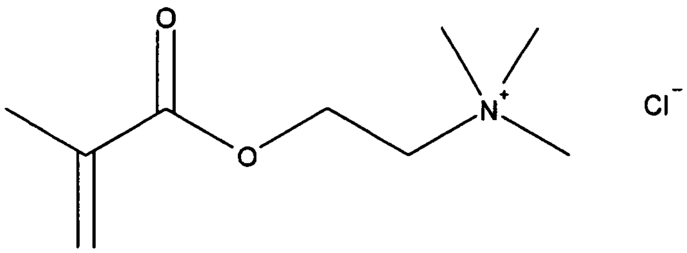

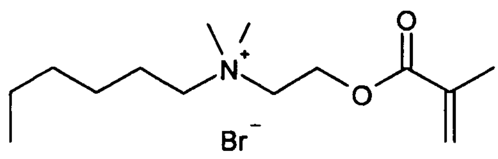

- la N,N-dimethyl-N-(2-methacryloyloxyethyl)-N-(3-sulfopropyl) ammonium bétaïne; la N,N-dimethyl-N-(3-methacrylamidopropyl)-N-(3-sulfopropyl) ammonium bétaïne, la 1-(3-sulfopropyl)-2-vinylpyridinium bétaïne et la 2-méthacryloyloxyéthylphosphorylcholine.

**8.** Dispersion selon l'une des revendications précédentes, dans laquelle le monomère cationique hydrophile est choisi parmi, seul ou en mélange :

- le (méth)acrylamide de diméthylaminopropyle, le (méth)acrylamide de diméthylaminoéthyle,
- le (méth)acrylate de diéthylaminoéthyle, le (méth)acrylate de diméthylaminoéthyle, le (méth)acrylate de morpholinoéthyle;
- la vinylimidazole, la vinylpyridine,

et les monomères ci-après :

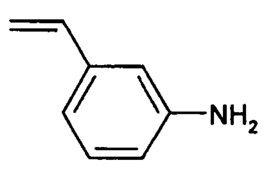

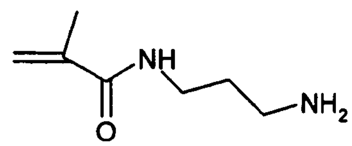

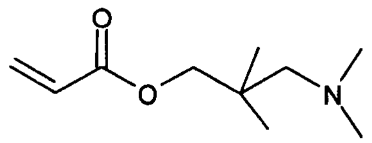

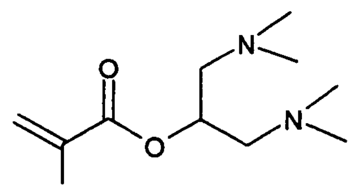

**9.** Dispersion selon l'une des revendications précédentes, dans laquelle le polymère éthylénique comprend 100% en poids de monomère cationique hydrophile ou un mélange de tels monomères cationiques hydrophiles, par rapport au poids total des monomères initiaux.

**10.** Dispersion selon l'une des revendications 1 à 8, dans laquelle le polymère éthylénique comprend un ou plusieurs monomères additionnels, présents à raison de 0,01 à 50% en poids par rapport au poids total de monomères.

**11.** Dispersion selon l'une des revendications précédentes, dans laquelle le monomère additionnel est choisi parmi, seul ou en mélange :

- (i) les esters de l'acide (méth)acrylique de formule $CH_2$=CHCOOR4 ou $CH_2$=C($CH_3$)COOR4 avec R4 représentant une chaîne carbonée, ayant 1 à 32 atomes de carbone, linéaire, cyclique ou ramifiée, saturée ou insaturée, comprenant ou non intercalé(s) un ou plusieurs hétéroatomes choisis parmi O, N, S; et/ou substituée ou non par un ou plusieurs substituants choisis parmi -OH et les atomes d'halogène (Cl, Br, I et F).
- (ii) les (méth)acrylamides de formule $CH_2$=CHCONR5R'5 ou $CH_2$=C($CH_3$)CONR5R'5 dans lesquelles R5 et R'5, identiques ou différents, représentent un atome d'hydrogène ou une chaîne carbonée, ayant 6 à 28 atomes de carbone, linéaire, cyclique ou ramifiée, saturée ou insaturée, ou aromatique, comprenant ou non intercalé (s) un ou plusieurs hétéroatomes choisis parmi O, N, S; et/ou substituée ou non par un ou plusieurs substituants choisis parmi -OH et les atomes d'halogène (Cl, Br, I et F).
- (iii) les esters vinyliques de formule $CH_2$=CH-OCO-R6 avec R6 représentant une chaîne carbonée, ayant 1 à 12 atomes de carbone, linéaire ou ramifiée, saturée ou insaturée;
- (iv) les éthers de vinyle de formule $CH_2$=CHOR7 avec R7 représentant une chaîne carbonée, ayant 1 à 12 atomes de carbone, linéaire ou ramifiée, saturée ou insaturée;

- (v) les composés vinyliques de formule $CH_2$=CHR8 dans laquelle R8 est
- un groupe hydroxyle;
- un groupe alkyle linéaire ou ramifié, comprenant 1 à 25 atomes de carbone, dans lequel se trouve(nt) ou non intercalé(s) un ou plusieurs hétéroatomes choisis parmi O, N, S et P; ledit groupe alkyle étant ou non substitué par un ou plusieurs substituants choisis parmi -OH et les atomes d'halogène (Cl, Br, I et F);
- un groupe cycloalkyle en $C_3$ à $C_8$,
- un groupe aryle en $C_6$ à $C_{20}$,
- un groupe aralkyle en $C_7$ à $C_{30}$ (groupe alkyle en $C_1$ à $C_4$),
- un groupe hétérocyclique de 4 à 12 chaînons contenant un ou plusieurs hétéroatomes choisis parmi O, N, et S,
- un groupe hétérocycloalkyle (alkyle de 1 à 4 carbones),

lesdits groupes cycloalkyle, aryle, aralkyle, hétérocyclique ou hétérocycloalkyle étant ou non substitué par un ou plusieurs substituants choisis parmi le groupe hydroxyle, les atomes d'halogène, et les groupes alkyles en C1-C4, linéaires ou ramifiés dans lesquels se trouve(nt) ou non intercalé(s) un ou plusieurs hétéroatomes choisis parmi O, N, S et P, lesdits groupes alkyle étant ou non substitués par un ou plusieurs substituants choisis parmi -OH, les atomes d'halogène (Cl, Br, I et F).

- (vi) des monomères multifonctionnels,;
- (vii) l'acide (méth)acrylique; l'anhydride maléique; l'acide crotonique, l'acide itaconique, l'acide fumarique, l'acide maléique, l'acide diacrylique, l'acide diméthylfumarique, l'acide citraconique, l'acide acrylamidopropanesulfonique, l'acide 2-acrylamido2-méthylpropanesulfonique, l'acide styrènesulfonique, l'acide vinylbenzoïque, l'acide vinylphosphorique, l'acide vinylsulfonique, l'acide vinylbenzène sulfonique, l'acide acrylamidoglycolique de formule CH2=CH-CONHCH(OH)COOH, l'acide vinylphosphonique; le (méth)acrylate de sulfopropyle, le (méth)acrylate de sulfoéthyle, la vinylméthylsulfone, le 2-(méthacryloyloxy)éthylphosphate de formule $CH_2=C(CH_3)COOC_2H_4OP(O)(OH)_2$; ainsi que leurs sels; et leurs mélanges.
- (viii) les (méth)acrylates de poly(éthylène glycol), les (méth)acrylates d'alkylpoly(éthylène glycol), et plus particulièrement les méthacrylates de méthylpoly(éthylène glycol).

**12.** Dispersion selon la revendication 11, dans laquelle le monomère additionnel est choisi parmi les (méth)acrylates et (méth)acrylamides de méthyle, d'éthyle, de propyle, de n-butyle, d'isobutyle, de 2-éthylhexyle, de tertiobutyle, d'isooctyle, de décyle, de myristyle, de stéaryle; ou le styrène, l'éthylhexylvinylether, le dodécylvinyléther, l'hexanoate de vinyle; l'acrylate de 2-hydroxyéthyle et le méthacrylate de poly(éthylene glycol), ainsi que leurs mélanges.

**13.** Dispersion selon l'une des revendications précédentes, dans laquelle le composé liquide non aqueux ayant un paramètre de solubilité global selon l'espace de solubilité de HANSEN inférieur ou égal à 20 $(MPa)^{1/2}$, est choisi parmi, seul ou en mélange, les huiles naturelles ou synthétiques, carbonées, hydrocarbonées, fluorées et/ou siliconées, ramifiées ou non.

**14.** Dispersion selon l'une des revendications précédentes, dans laquelle le composé liquide non aqueux est choisi parmi :

- les huiles végétales formées par des esters d'acides gras et de polyols; les esters dérivés d'acides ou d'alcools à longue chaîne en C6-C20; les esters de formule RCOOR' dans laquelle R représente le reste d'un acide gras supérieur comportant de 7 à 19 atomes de carbone et R' représente une chaîne hydrocarbonée comportant de 3 à 20 atomes de carbone;
- les hydrocarbures ;
- les huiles de silicone substituées ou non par des groupements aliphatiques et/ou aromatiques, fluorés ou non, ou par des groupements fonctionnels; et les huiles siliconées volatiles;
- les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.
- les monoalcools gras aliphatiques ayant au moins 6 atomes de carbone, la chaîne hydrocarbonée ne comportant pas de groupement de substitution.

**15.** Dispersion selon l'une des revendications précédentes, dans laquelle ledit composé liquide non aqueux est choisi parmi les huiles siliconées volatiles, cycliques ou linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone; ainsi que leurs mélanges.

**16.** Dispersion selon l'une des revendications précédentes, dans laquelle l'agent stabilisant est choisi parmi les polymères séquencés, les polymères greffés et/ou les polymères statistiques, seul ou en mélange.

**17.** Dispersion selon l'une des revendications précédentes, dans laquelle l'agent stabilisant est présent à raison de 0,1 à 30% en poids par rapport au poids du mélange initial de monomères.

**18.** Dispersion selon l'une des revendications précédentes, dans laquelle l'agent stabilisant est choisi parmi :

- les polymères siliconés greffés avec une chaîne hydrocarbonée et les polymères hydrocarbonés greffés avec une chaîne siliconée.
- les copolymères greffés ayant un squelette insoluble de type polyacrylique avec des greffons solubles de type acide poly (12-hydroxy stéarique).
- les copolymères blocs greffés ou séquencés comprenant au moins un bloc de type polyorganosiloxane et au moins un bloc d'un polymère (i) issu de la polymérisation radicalaire ou (ii) issu d'une polycondensation, ou leur mélange, ledit copolymère pouvant comporter des entités fluorées;
- les copolymères blocs, greffés ou séquencés, de (méth)acrylates d'alkyle en C1-C4 et de (méth)acrylates d'alkyle en C8-C30;
- les copolymères blocs greffés ou séquencés comprenant au moins un bloc résultant de la polymérisation de monomère éthylénique, à une ou plusieurs liaisons éthyléniques éventuellement conjuguées; et au moins un bloc de polymère issu de la polymérisation radicalaire autre que diène;
- les alkyl-dimethicones dans lesquels le groupement alkyl comprend 6 à 32 atomes de carbone;
- les dimethiconol ester de formule :

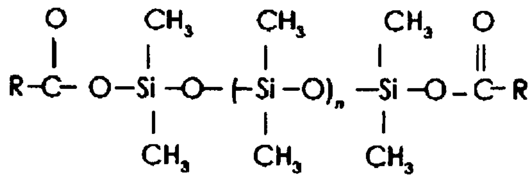

dans laquelle R est un radical alkyle ayant 6 à 32 atomes de carbone,
- les alkylamidoamines ayant notamment 6 à 60 atomes de carbone,
- les copolymères comprenant au moins une partie polyorganosiloxane et des groupements fluorés, notamment de formule :

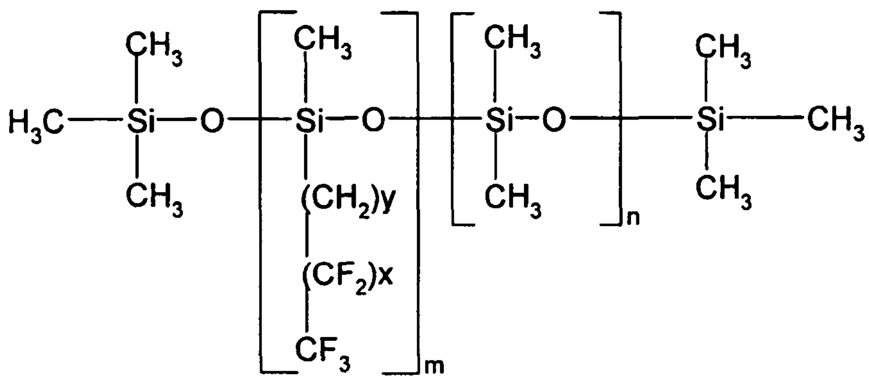

dans laquelle x est un entier compris entre 3 et 12; y est un entier compris entre 2 et 6; et m et n sont tels que le poids moléculaire du composé est compris entre 5000 et 15000.

**19.** Dispersion selon l'une des revendications précédentes, dans laquelle l'agent stabilisant est choisi parmi :

- les silicones fluorées ou fluorosilicones de formule :

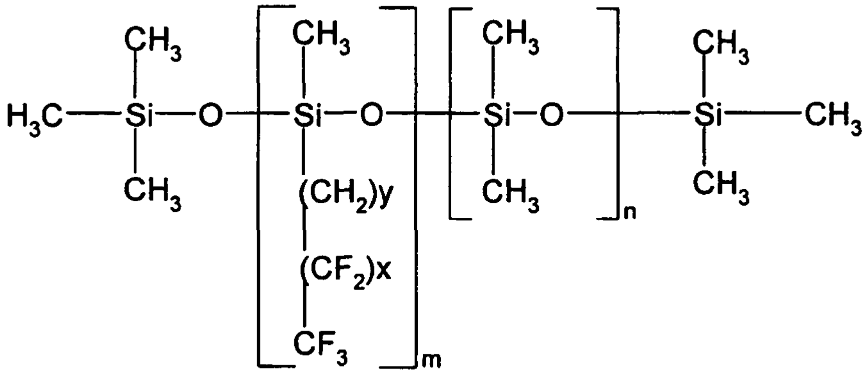

dans laquelle x=8, y = 2 ou 3, et m et n sont tels que le poids moléculaire du composé est compris entre 5000 et 15000; et
- les copolymères blocs greffés ou séquencés comprenant au moins un bloc de type polyorganosiloxane et au moins un bloc d'un polymère issu d'une polycondensation, notamment de type polyéther, polyester ou polyamide; en particulier, un bloc polyéther de type polyalkylène en $C_2$-$C_{18}$, notamment polyoxyéthylène et/ou polyoxypropylène.

**20.** Composition cosmétique ou pharmaceutique comprenant au moins une dispersion telle que définie selon l'une des revendications 1 à 19, dans un milieu cosmétiquement ou pharmaceutiquement acceptable qui comprend au moins un constituant choisi parmi les cires, les huiles, les gommes et/ou les corps gras pâteux, d'origine végétale, animale, minérale ou de synthèse, voire siliconés, et leurs mélanges; une ou plusieurs matières colorantes choisies parmi les composés pulvérulents et/ou les colorants liposolubles ou hydrosolubles; les antioxydants, les parfums, les huiles essentielles, les conservateurs, les actifs cosmétiques, les hydratants, les vitamines, les acides gras essentiels, les céramides, les filtres solaires, les tensioactifs, les polymères, les épaississants, les gélifiants.

**21.** Composition selon la revendication 20, se présentant sous la forme d'une suspension, une dispersion; une solution huileuse éventuellement épaissie voire gélifiée; une émulsion huile-dans-eau, eau-dans-huile, ou multiple; un gel ou une mousse; un gel huileux ou émulsionné; une dispersion de vésicules; une lotion biphase ou multiphase; un spray; d'une lotion, d'une crème, d'une pommade, d'une pâte souple, d'un onguent, d'un solide coulé ou moulé, ou encore de solide compacté.

**22.** Composition selon l'une des revendications 20 à 21, se présentant sous la forme d'un produit de soin et/ou de maquillage de la peau du corps ou du visage, des lèvres et des cheveux, d'un produit solaire ou autobronzant; d'un produit capillaire.

**23.** Composition selon l'une des revendications 20 à 22, se présentant sous la forme d'un shampooing, gel, lotion de mise en plis, lotion pour le brushing, composition de fixation et de coiffage telle que les laques, des mousses ou des sprays; d'un gel-douche, d'un bain moussant; d'un après-shampooing à rincer ou non, d'une composition pour permanente, défrisage, coloration ou décoloration; d'une compositions à rincer, à appliquer avant ou après une coloration, une décoloration, une permanente ou un défrisage ou encore entre les deux étapes d'une permanente ou d'un défrisage; de compositions lavantes pour la peau, et en particulier sous la forme de solutions ou de gels pour le bain ou la douche ou de produits démaquillants; de lotions aqueuses ou hydroalcooliques pour le soin de la peau et/ou des cheveux; de produit de soin du cheveu (masque capillaire).

**24.** Procédé de traitement cosmétique des matières kératiniques, comprenant l'application sur lesdites matières d'une composition cosmétique telle que définie à l'une des revendications 20 à 23.

## Claims

**1.** Dispersion of particles of at least one ethylenic polymer, which particles are stabilized at the surface by a stabilizing agent, in a nonaqueous medium composed of at least one nonaqueous compound which is liquid at 25°C and which has an overall solubility parameter, according to the Hansen solubility space, of less than or equal to 20 $(MPa)^{1/2}$, or a mixture of such compounds, **characterized in that** the said ethylenic polymer comprises from 50 to 100% by

weight of hydrophilic cationic monomer having a log p of less than or equal to 2, or of a mixture of such monomers, with respect to the total weight of monomers.

**2.** Dispersion according to Claim 1, in which the polymer comprises from 50 to 100% by weight of hydrophilic cationic monomer, alone or as a mixture, with respect tc the total weight of starting monomers.

**3.** Dispersion according to either of the preceding claims, in which the hydrophilic cationic monomer exhibits a log p value of between -10 and 2.

**4.** Dispersion according to one of the preceding claims, in which the hydrophilic cationic monomer is chosen, alone or as a mixture, from the monomers of following formulae (I) and (II), and their salts:

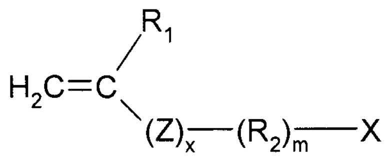

(I)

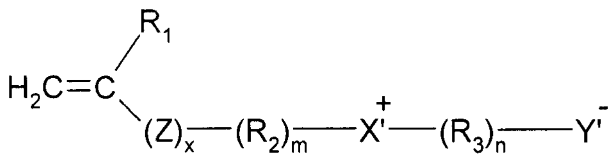

(II)

in which:

- $R_1$ is a hydrogen atom or a linear or branched hydrocarbon radical of $C_pH_{2p+1}$ type, with p being an integer between 1 and 12 inclusive;
- Z is a divalent group chosen from -COO-, -CONH-,
- $CONCH_3$-, -OCO-, -0-, $-SO_2$- -CO-O-CO- or $-CO-CH_2-CO-$;
- x is 0 or 1;
- $R_2$ and $R_3$ are, independently of one another, a saturated or unsaturated, or aromatic, linear, branched or cyclic, divalent carbon-comprising radical of 1 to 30 carbon atoms, optionally comprising from 1 to 18 heteroatoms chosen from 0, N, S, F, Si and P;
- m is 0 or 1;
- n is between 1 and 100;
- X is:

(a) a guanidino group or an amidino group; or
(b) a group of formula $-N(R_6)(R_7)$ with $R_6$ and $R_7$ representing, independently of one another, (i) a hydrogen atom, (ii) a saturated or unsaturated, or aromatic, linear, branched or cyclic alkyl group comprising from 1 to 18 carbon atoms, optionally comprising from 1 to 10 heteroatoms chosen from O, N, S, F, Si and P; or else (iii) $R_6$ and $R_7$ form, with the nitrogen atom, a saturated or unsaturated, or aromatic, ring of formula:

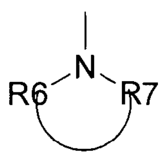

comprising in total from 5 to 8 atoms; the said first ring optionally being fused to one or more other saturated or unsaturated, or aromatic, rings each comprising from 5 to 7 atoms;
(c) a ring:

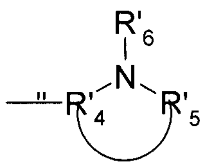

in which $R'_4$ and $R'_5$ form, with the nitrogen atom, a saturated or unsaturated, or aromatic, ring comprising in total from 5 to 8 atoms; the said ring optionally being fused to one or more other saturated or unsaturated, or aromatic, rings each comprising from 5 to 7 atoms; and $R'_6$ is chosen from H, $-CH_3$ and $-C_2H_5$;

- $X'^+$ is a divalent group of formula $-N^+(R_6)(R_7)-$ with $R_6$ and $R_7$ representing, independently of one another, (i) a hydrogen atom, (ii) a linear, branched or cyclic, or aromatic, alkyl group comprising from 1 to 25 carbon atoms, optionally comprising from 1 to 20 heteroatoms chosen from O, N, S and P; or (iii) $R_6$ and $R_7$ form, with the nitrogen atom, a saturated or unsaturated, or aromatic, ring of formula:

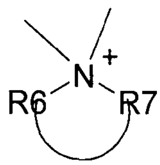

comprising in total from 5 to 8 atoms; the said first ring optionally being fused to one or more other saturated or unsaturated, or aromatic, rings each comprising from 5 to 7 atoms;
or else $X'^+$ is a divalent ring of formula:

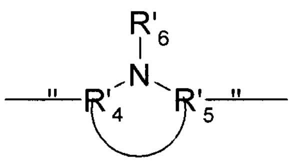

in which $R'_4$ and $R'_5$ form, with the nitrogen atom, a saturated or unsaturated, or aromatic, ring comprising in total from 5 to 8 atoms; the said ring optionally being fused to one or more other saturated or unsaturated, or aromatic, rings each comprising from 5 to 7 atoms; and $R'_6$ is chosen from H, $-CH_3$ and $-C_2H_5$;
- $Y'^-$ is a group chosen from $-COO^-$, $-SO_3^-$, $-OSO_3^-$, $-PO_3^{2-}$ and $-OPO_3^{2-}$.

**5.** Dispersion according to Claim 4, in which X is chosen from:

- a group of formula $-N(R_6)(R_7)$ with $R_6$ and $R_7$ representing, independently of one another, (i) a hydrogen atom, (ii) a saturated or unsaturated, or aromatic, linear, branched or cyclic alkyl group comprising from 1 to 18 carbon atoms, or (iii) $R_6$ and $R_7$ form, with the nitrogen atom, a saturated or unsaturated, or aromatic, ring of formula:

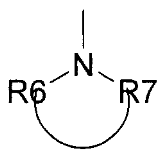

comprising in total from 5 to 7 atoms;
- a ring:

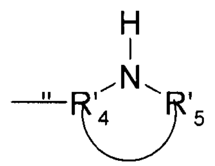

in which R'$_4$ and R'$_5$ form, with the nitrogen atom, a saturated or unsaturated, or aromatic, ring comprising from 5 to 7 atoms.

**6.** Dispersion according to either of Claims 4 and 5, in which X is chosen from an $NH_2$, $N(CH_3)_2$, pyridinyl, imidazolyl, piperidinyl, piperazinyl or morpholinyl group.

**7.** Dispersion according to one of the preceding claims, in which the hydrophilic cationic monomer is chosen, alone or as a mixture, from:

- dimethylaminopropyl(meth)acrylamide, dimethylaminoethyl(meth)acrylamide or diethylaminopropyl (meth)-acrylamide;
- diethylaminoethyl (meth)acrylate, dimethylaminoethyl (meth)acrylate, morpholinoethyl (meth)acrylate or tertbutylaminoethyl (meth)acrylate;
- vinylimidazole, vinylpyridine, vinylamine, and the following monomers:

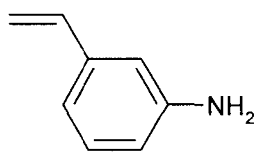

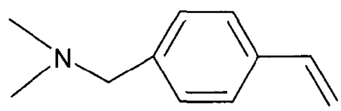

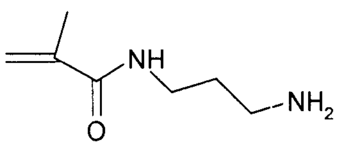

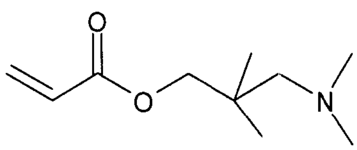

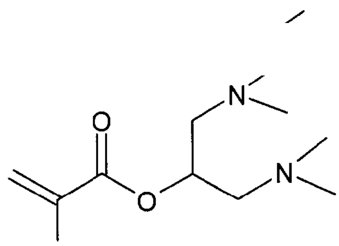

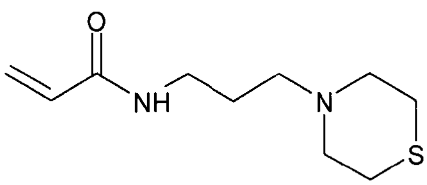

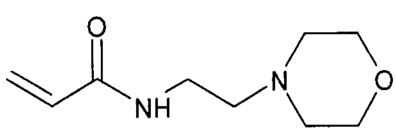

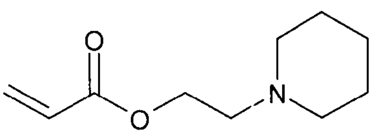
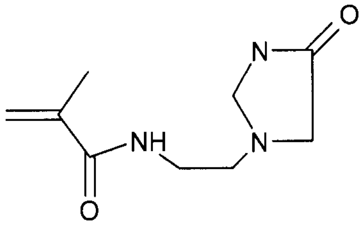
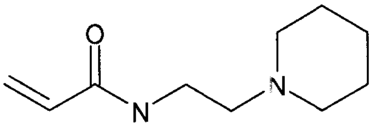
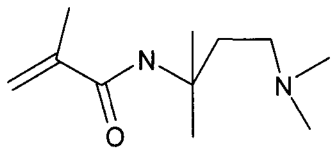
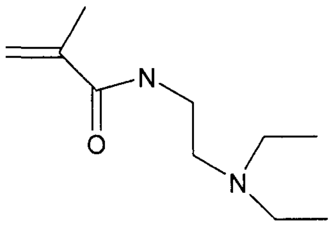
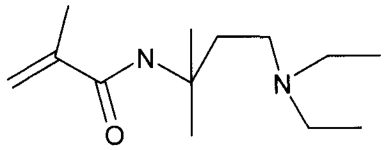
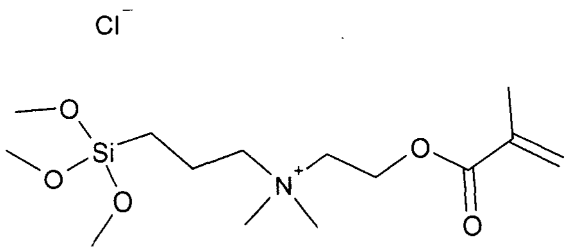
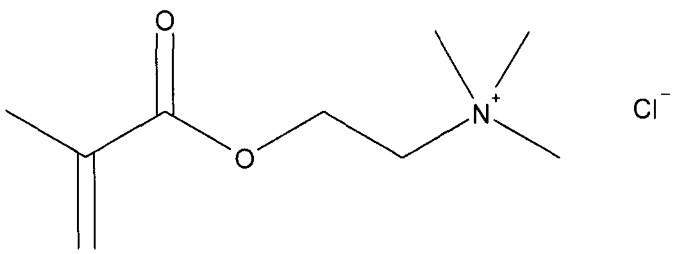
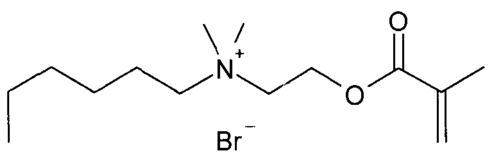

- N,N-dimethyl-N-(2-methacryloyloxyethyl)-N-(3-sulphopropyl)ammonium betaine, N,N-dimethyl-N-(3-methacrylamidopropyl)-N-(3-sulphopropyl)ammonium betaine, 1-(3-sulphopropyl)-2-vinylpyridinium betaine and 2-methacryloyloxyethyl phosphorylcholine.

**8.** Dispersion according to one of the preceding claims, in which the hydrophilic cationic monomer is chosen, alone or as a mixture, from:

- dimethylaminopropyl(meth)acrylamide or dimethylaminoethyl(meth)acrylamide,

- diethylaminoethyl (meth)acrylate, dimethylaminoethyl (meth)acrylate or morpholinoethyl (meth)acrylate,
- vinylimidazole or vinylpyridine,

and the following monomers:

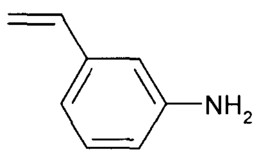

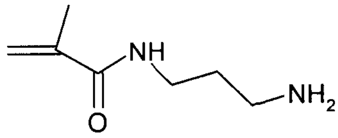

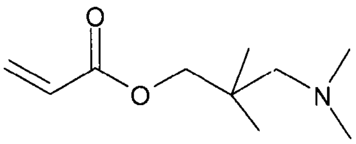

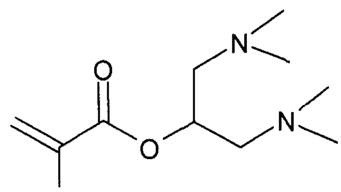

**9.** Dispersion according to one of the preceding claims, in which the ethylenic polymer comprises 100% by weight of hydrophilic cationic monomer or a mixture of such hydrophilic cationic monomers, with respect to the total weight of the starting monomers.

**10.** Dispersion according to one of Claims 1 to 8, in which the ethylenic polymer comprises one or more additional monomers present in a proportion of 0.01 to 50% by weight, with respect to the total weight of monomers.

**11.** Dispersion according to one of the preceding claims, in which the additional monomer is chosen, alone or as a mixture, from:

- (i) (meth)acrylic acid esters of formula $CH_2=CHCOOR_4$ or $CH_2=C(CH_3)COOR_4$ with $R_4$ representing a saturated or unsaturated, linear, cyclic or branched carbon-comprising chain, having from 1 to 32 carbon atoms and optionally comprising, inserted, one or more heteroatoms chosen from O, N, S and/or optionally substituted by one or more substituents chosen from -OH and halogen atoms (Cl, Br, I and F);
- (ii) (meth)acrylamides of formula $CH_2=CHCONR_5R'_5$ or $CH_2=C(CH_3)CONR_5R'_5$ in which $R_5$ and $R'_5$, which are identical or different, represent a hydrogen atom or a saturated or unsaturated, or aromatic, linear, cyclic or branched carbon-comprising chain, having from 6 to 28 carbon atoms and optionally comprising, inserted, one or more heteroatoms chosen from O, N and S; and/or optionally substituted by one or more substituents chosen from -OH and halogen atoms (Cl, Br, I and F);
- (iii) vinyl esters of formula $CH_2=CH\text{-}OCO\text{-}R_6$ with $R_6$ representing a saturated or unsaturated, linear or branched, carbon-comprising chain, having from 1 to 12 carbon atoms;
- (iv) vinyl ethers of formula $CH_2=CHOR_7$ with $R_7$ representing a saturated or unsaturated, linear or branched carbon-comprising chain, having from 1 to 12 carbon atoms;
- (v) vinyl compounds of formula $CH_2=CHR_8$ in which $R_8$ is
- a hydroxyl group;
- a linear or branched alkyl group comprising from 1 to 25 carbon atoms in which one or more heteroatoms chosen from O, N, S and P is/are optionally inserted; the said alkyl group optionally being substituted by one or more substituents chosen from -OH and halogen atoms (Cl, Br, I and F) ;
- a $C_3$ to $C_8$ cycloalkyl group,
- a $C_6$ to $C_{20}$ aryl group,
- a $C_7$ to $C_{30}$ aralkyl group ($C_1$ to $C_4$ alkyl group),
- a 4- to 12-membered heterocyclic group comprising one or more heteroatoms chosen from O, N and S,
- a heterocycloalkyl group (alkyl group of 1 to 4 carbons),

the said cycloalkyl, aryl, aralkyl, heterocyclic or heterocycloalkyl groups optionally being substituted by one or more substituents chosen from the hydroxyl group, halogen atoms and linear or branched $C_1$-$C_4$ alkyl groups in which one or more heteroatoms chosen from O, N, S and P is/are optionally inserted, the said alkyl groups optionally being substituted by one or more substituents chosen from -OH and halogen atoms (Cl, Br, I and F) ;
- (vi) multifunctional monomers;
- (vii) (meth)acrylic acid, maleic anhydride, crotonic acid, itaconic acid, fumaric acid, maleic acid, diacrylic acid,

dimethylfumaric acid, citraconic acid, acrylamidopropanesulphonic acid, 2-acrylamido-2-methylpropanesulphonic acid, styrenesulphonic acid, vinylbenzoic acid, vinylphosphoric acid, vinylsulphonic acid, vinylbenzenesulphonic acid, acrylamidoglycolic acid of formula $CH_2=C_H-CONHCH(OH)COOH$, vinylphosphonic acid, sulphopropyl (meth)acrylate, sulphoethyl (meth)acrylate, vinyl methyl sulphone, 2-(methacryloyloxy)ethyl phosphate of formula $CH_2=C(CH_3)COOC_2H_4OP(O)(OH)_{2,}$ and their salts and their mixtures;
- (viii) poly(ethylene glycol) (meth)acrylates, alkyl poly(ethylene glycol) (meth)acrylates and more particularly methyl poly(ethylene glycol) methacrylates.

**12.** Dispersion according to Claim 11, in which the additional monomer is chosen from methyl, ethyl, propyl, n-butyl, isobutyl, 2-ethylhexyl, tert-butyl, isooctyl, decyl, myristyl or stearyl (meth)acrylates and methyl-, ethyl-, propyl-, (n-butyl)-, isobutyl-, (2-ethylhexyl)-, (tert-butyl)-, isooctyl-, decyl-, myristyl- or stearyl(meth)acrylamides; or styrene, ethylhexyl vinyl ether, dodecyl vinyl ether, vinyl hexanoate; 2-hydroxyethyl acrylate and poly(ethylene glycol) methacrylate, and their mixtures.

**13.** Dispersion according to one of the preceding claims, in which the nonaqueous liquid compound having an overall solubility parameter according to the Hansen solubility space of less than or equal to 20 $(MPa)^{1/2}$ is chosen, alone or as a mixture, from optionally branched, carbon-comprising, hydrocarbon, fluorinated and/or silicone natural or synthetic oils.

**14.** Dispersion according to one of the preceding claims, in which the nonaqueous liquid compound is chosen from:

- vegetable oils formed by esters of fatty acids and of polyols; esters derived from long-chain $C_6$-$C_{20}$ acids or alcohols; esters of formula RCOOR' in which R represents the residue of a higher fatty acid comprising from 7 to 19 carbon atoms and R' represents a hydrocarbon chain comprising from 3 to 20 carbon atoms;
- hydrocarbons;
- silicone oils, optionally substituted by optionally fluorinated aliphatic and/or aromatic groups or by functional groups; and volatile silicone oils;
- solvents, alone or as a mixture, chosen from linear, branched or cyclic esters having from 6 to 30 carbon atoms, ethers having from 6 to 30 carbon atoms and ketones having from 6 to 30 carbon atoms;
- fatty aliphatic monoalcohols having at least 6 carbon atoms, the hydrocarbon chain not comprising a substituent group.

**15.** Dispersion according to one of the preceding claims, in which the said nonaqueous liquid compound is chosen from cyclic or linear volatile silicone oils, and/or esters of formula RCOOR' in which R represents the residue of a higher fatty acid comprising from 7 to 19 carbon atoms and R' represents a hydrocarbon chain comprising from 3 to 20 carbon atoms; and their mixtures.

**16.** Dispersion according to one of the preceding claims, in which the stabilizing agent is chosen from sequential polymers, grafted polymers and/or random polymers, alone or as a blend.

**17.** Dispersion according to one of the preceding claims, in which the stabilizing agent is present in a proportion of 0.1 to 30% by weight, with respect to the weight of the starting mixture of monomers.

**18.** Dispersion according to one of the preceding claims, in which the stabilizing agent is chosen from:

- silicone polymers grafted with a hydrocarbon chain and hydrocarbon polymers grafted with a silicone chain;
- grafted copolymers having an insoluble backbone of polyacrylic type with soluble grafts of poly(12-hydroxystearic acid) type;
- grafted or sequential block copolymers comprising at least one block of polyorganosiloxane type and at least one block of a polymer (i) resulting from radical polymerization or (ii) resulting from polycondensation, or their blend, it being possible for the said copolymer to comprise fluorinated entities;
- grafted or sequential block copolymers of $C_1$-$C_4$ alkyl (meth)acrylates and of $C_8$-$C_{30}$ alkyl (meth)acrylates,;
- grafted or sequential block copolymers comprising at least one block resulting from the polymerization of ethylenic monomer, comprising one or more optionally conjugated ethylenic bonds; and at least one block of polymer resulting from radical polymerization other than dienes;
- alkyl dimethicones in which the alkyl group comprises 6 to 32 carbon atoms;
- dimethiconol esters of formula:

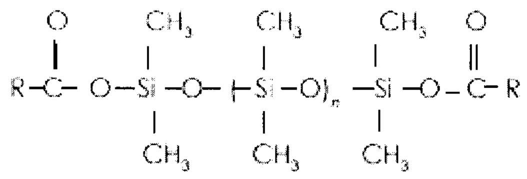

in which R is an alkyl radical having from 6 to 32 carbon atoms;
- alkylamidoamines having in particular from 6 to 60 carbon atoms;
- copolymers comprising at least one polyorganosiloxane part and fluorinated groups, in particular of formula:

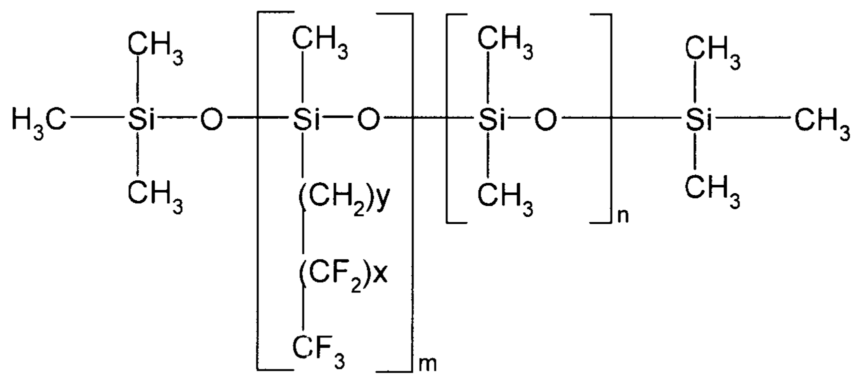

in which x is an integer between 3 and 12; y is an integer between 2 and 6; and m and n are such that the molecular weight of the compound is between 5000 and 15 000.

**19.** Dispersion according to one of the preceding claims, in which the stabilizing agent is chosen from:

- fluorinated silicones or fluorosilicones of formula:

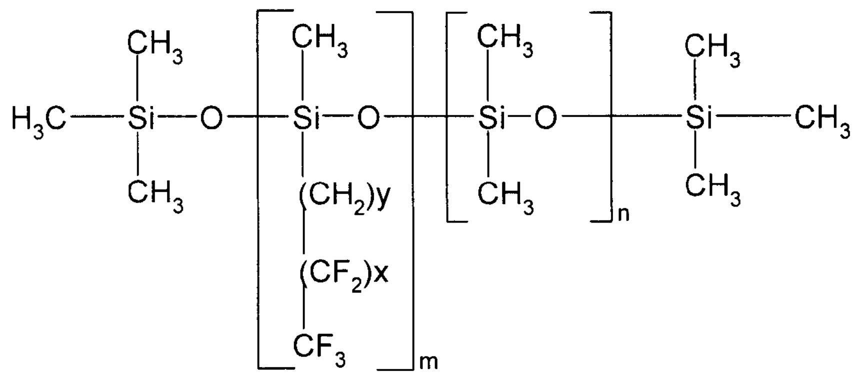

in which x = 8, y = 2 or 3, and m and n are such that the molecular weight of the compound is between 5000 and 15 000; and
- grafted or sequential block copolymers comprising at least one block of polyorganosiloxane type and at least one block of a polymer resulting from polycondensation, in particular of polyether, polyester or polyamide type; especially a polyether block of polyoxy-($C_2$-$C_{18}$)alkylene type, in particular polyoxyethylene and/or polyoxypropylene type.

**20.** Cosmetic or pharmaceutical composition comprising at least one dispersion as defined according to one of Claims 1 to 19, in a cosmetically or pharmaceutically acceptable medium which comprises at least one constituent chosen from waxes, oils, gums and/or pasty fatty substances of vegetable, animal, mineral or synthetic, indeed even silicone, origin, and their mixtures; one cr more colouring materials chosen from pulverulent compounds and/or fat-soluble or watersoluble dyes; antioxidants, fragrances, essential oils, preservatives, cosmetic active principles, moisturizing

agents, vitamins, essential fatty acids, ceramides, sunscreens, surfactants, polymers, thickeners or gelling agents.

**21.** Composition according to Claim 20, which is provided in the form of a suspension, a dispersion; an optionally thickened, indeed even gelled, oily solution; an oil-in-water, water-in-oil or multiple emulsion; a gel or a foam; an oily or emulsified gel; a dispersion of vesicles; a two-phase or multiphase lotion; a spray; of a lotion, of a cream, of an ointment, of a soft paste, of a salve, of a cast or moulded solid, or else of a compacted solid.

**22.** Composition according to either of Claims 20 and 21, which is provided in the form of a product for caring for and/or making up the skin of the body or face, the lips and the hair, of an antisun or selftanning product; of a hair product.

**23.** Composition according to one of Claims 20 to 22, which is provided in the form of a shampoo, gel, hairsetting lotion, blow-drying lotion, or fixing and styling composition, such as lacquers, foams or sprays; of a shower gel or of a foam bath; of a rinse-out or leave-in conditioner, of a perming, hair-straightening, dyeing or bleaching composition; of a rinse-out composition, to be applied before or after dyeing, bleaching, perming or hair straightening or else between the two stages of a perming or hair straightening; of washing compositions for the skin and in particular in the form of solutions or gels for the bath or shower or of make-up removers; of aqueous or aqueous/alcoholic lotions for caring for the skin and/or hair; or of a hair care product (hair mask).

**24.** Method for the cosmetic treatment of keratinous substances, comprising the application, to the said substances, of a cosmetic composition as defined in one of Claims 20 to 23.

**Patentansprüche**

**1.** Dispersion von Teilchen mindestens eines ethylenischen Polymers, die an der Oberfläche durch ein Stabilisierungsmittel stabilisiert sind, in einem nichtwässrigen Medium, das aus mindestens einer nichtwässrigen Verbindung, die bei 25°C flüssig ist und einen Gesamtlöslichkeitsparameter gemäß dem Löslichkeitsraum nach Hansen kleiner gleich 20 $(MPa)^{1/2}$ aufweist, oder einer Mischung derartiger Verbindungen besteht; **dadurch gekennzeichnet, dass** das ethylenische Polymer 50 bis 100 Gew.-% hydrophiles kationisches Monomer mit einem log-p-Wert kleiner gleich 2 oder einer Mischung derartiger Monomere, bezogen auf das Gesamtgewicht der Monomere, umfasst.

**2.** Dispersion nach Anspruch 1, wobei das Polymer 50 bis 100 Gew.-% hydrophiles kationisches Monomer allein oder als Mischung, bezogen auf das Gesamtgewicht der Startmonomere, umfasst.

**3.** Dispersion nach einem der vorhergehenden Ansprüche, wobei das hydrophile kationische Monomer einen log-p-Wert zwischen -10 und 2 aufweist.

**4.** Dispersion nach einem der vorhergehenden Ansprüche, wobei das hydrophile kationische Monomer unter Monomeren der folgenden Formeln (I) und (II) sowie Salzen davon allein oder als Mischung ausgewählt ist:

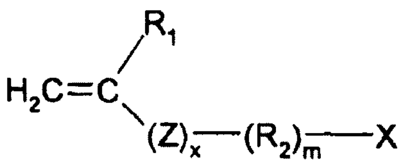

(I)

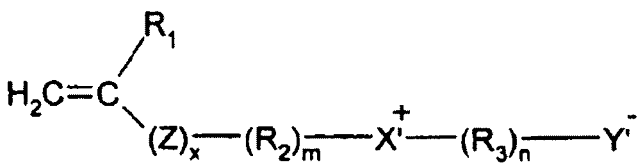

(II)

worin:

- R1 für ein Wasserstoffatom oder einen linearen oder verzweigten auf Kohlenwasserstoff basierenden Rest vom Typ $CpH_{2p+1}$, wobei p eine ganze Zahl zwischen 1 und 12 einschließlich bedeutet, steht;

- Z für eine unter -COO-, -CONH-, $-CONCH_3-$, -OCO-,
- O-, $-SO_2-$, -CO-O-CO- oder $-CO-CH_2-CO-$ ausgewählte zweiwertige Gruppe steht;
- x für 0 oder 1 steht;
- R2 und R3 unabhängig voneinander für einen gesättigten oder ungesättigten, gegebenenfalls aromatischen, linearen, verzweigten oder cyclischen auf Kohlenstoff basierenden zweiwertigen Rest mit 1 bis 30 Kohlenstoffatomen, der gegebenenfalls 1 bis 18 unter 0, N, S, F, Si und P ausgewählte Heteroatome umfasst, stehen;
- m für 0 oder 1 steht;
- n zwischen 1 und 100 liegt;
- X für

(a) eine Guanidino- oder Amidinogruppe oder
(b) eine Gruppe der Formel $-N(R_6)(R_7)$, wobei R6 und R7 unabhängig voneinander (i) ein Wasserstoffatom oder (ii) eine lineare, verzweigte oder cyclische, gesättigte oder ungesättigte oder aromatische Alkylgruppe mit 1 bis 18 Kohlenstoffatomen, die gegebenenfalls 1 bis 10 unter O, N, S, F, Si und P ausgewählte Heteroatome umfasst, bedeuten; oder (iii) R6 und R7 mit dem Stickstoffatom einen Ring der Formel:

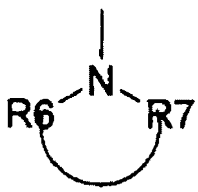

bilden, der gesättigt oder ungesättigt oder aromatisch ist und insgesamt 5 bis 8 Atome umfasst; wobei der erste Ring gegebenenfalls mit einem oder mehreren weiteren gesättigten oder ungesättigten oder aromatischen Ringen mit jeweils 5 bis 7 Atomen anelliert ist;
(c) einen Ring:

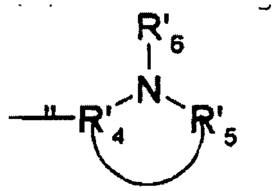

worin R'4 und R'5 mit dem Stickstoffatom einen gesättigten oder ungesättigten oder aromatischen Ring mit insgesamt 5 bis 8 Atomen bilden; wobei der Ring gegebenenfalls mit einem oder mehreren weiteren gesättigten oder ungesättigten oder aromatischen Ringen mit jeweils 5 bis 7 Atomen anelliert ist; und R'6 unter H, $-CH_3$ und $-C_2H_5$ ausgewählt ist;
steht;

- $X'^+$ für eine zweiwertige Gruppe der Formel
- $N^+(R_6)(R_7)$-, wobei R6 und R7 unabhängig voneinander (i) ein Wasserstoffatom oder (ii) eine lineare, verzweigte oder cyclische oder aromatische Alkylgruppe mit 1 bis 25 Kohlenstoffatomen, die gegebenenfalls 1 bis 20 unter O, N, S und P ausgewählte Heteroatome umfasst, bedeuten; oder (iii) R6 und R7 mit dem Stickstoffatom einen Ring der Formel:

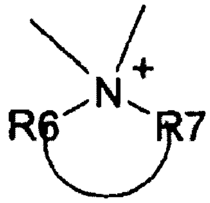

bilden, der gesättigt oder ungesättigt oder aromatisch ist und insgesamt 5 bis 8 Atome umfasst; wobei der erste

Ring gegebenenfalls mit einem oder mehreren weiteren gesättigten oder ungesättigten oder aromatischen Ringen mit jeweils 5 bis 7 Atomen anelliert ist;
oder $X'^{+}$ für einen zweiwertigen Ring der Formel:

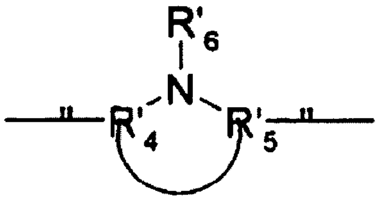

steht, worin R'4 und R'5 mit dem Stickstoffatom einen gesättigten oder ungesättigten oder aromatischen Ring mit insgesamt 5 bis 8 Atomen bilden; wobei der Ring gegebenenfalls mit einem oder mehreren weiteren gesättigten oder ungesättigten oder aromatischen Ringen mit jeweils 5 bis 7 Atomen anelliert ist; und R'6 unter H, $-CH_3$ und $-C_2H_5$ ausgewählt ist;
steht;

- $Y'^{-}$ für eine unter -COO-, $-SO_3^-$, $-OSO_3^-$, $-PO_3^{2-}$ und
- $OPO_3^{2-}$ ausgewählte Gruppe steht.

**5.** Dispersion nach Anspruch 4, wobei X unter:

- einer Gruppe der Formel $-N(R_6)(R_7)$, wobei R6 und R7 unabhängig voneinander (i) ein Wasserstoffatom oder (ii) eine lineare, verzweigte oder cyclische, gesättigte oder ungesättigte oder aromatische Alkylgruppe mit 1 bis 18 Kohlenstoffatomen bedeuten oder (iii) R6 und R7 mit dem Stickstoffatom einen Ring der Formel:

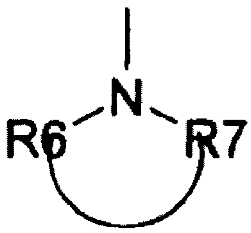

bilden, der gesättigt oder ungesättigt oder aromatisch ist und insgesamt 5 bis 7 Atome umfasst; und

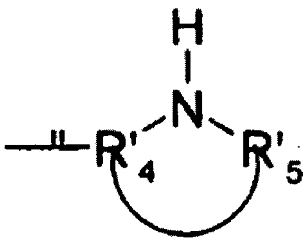

worin R'4 und R'5 mit dem Stickstoffatom einen gesättigten oder ungesättigten oder aromatischen Ring mit 5 bis 7 Atomen bilden;
ausgewählt ist.

**6.** Dispersion nach einem der Ansprüche 4 bis 5, wobei X unter einer $NH_2$-, $N(CH_3)_{2}$-, Pyridinyl-, Imidazolyl-, Piperidinyl-, Piperazinyl- oder Morpholingruppe ausgewählt ist.

**7.** Dispersion nach einem der vorhergehenden Ansprüche, wobei das hydrophile kationische Monomer unter

- Dimethylaminopropyl(meth)acrylamid, Dimethylaminoethyl(meth)acrylamid, Diethylaminopropyl-(meth)acrylamid;

- Diethylaminoethyl(meth)acrylat, Dimethylaminoethyl(meth)acrylat, Morpholinoethyl(meth)acrylat, tert.-Butylaminoethyl(meth)acrylat;
- Vinylimidazol, Vinylpyridin, Vinylamin und den nachstehenden Monomeren:

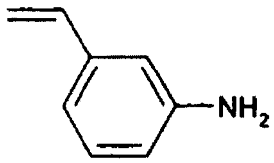

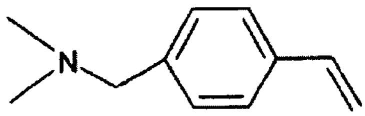

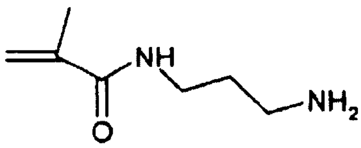

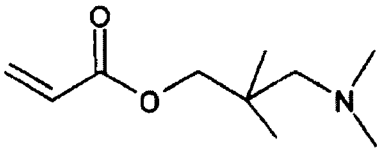

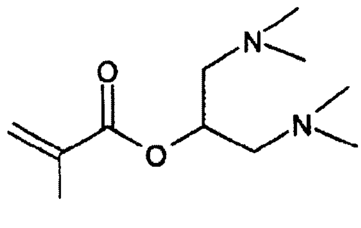

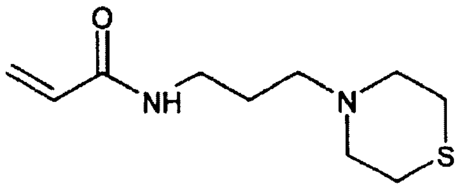

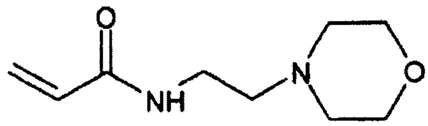

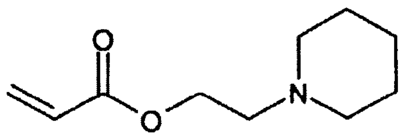

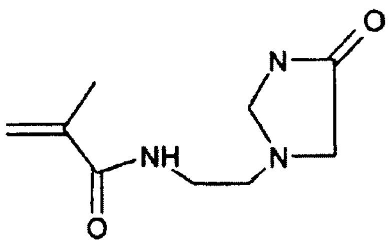

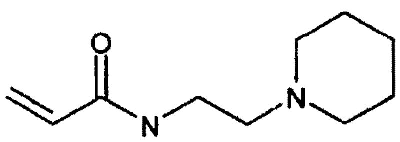

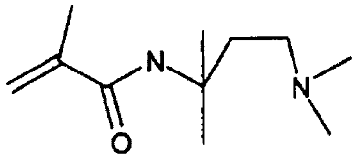

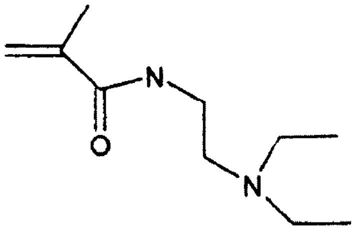

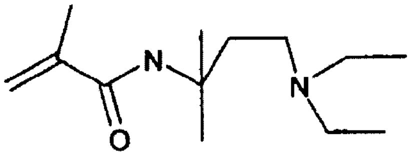

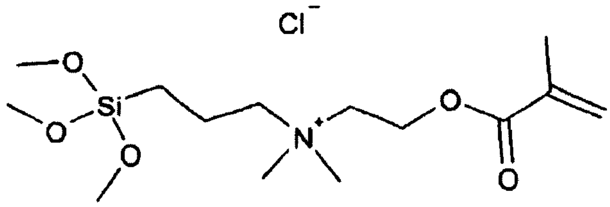

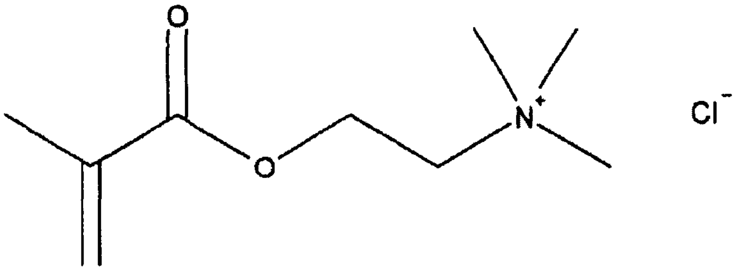

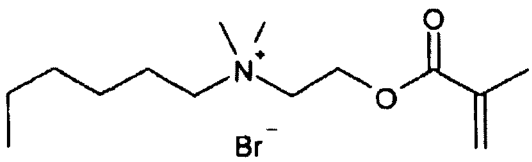

- N,N-Dimethyl-N-(2-methacryloyloxyethyl)-N-(3-sulfopropyl)ammoniumbetain; N,N-Dimethyl-N-(3-methacrylamidopropyl)-N-(3-sulfopropyl)ammonium-betain, 1-(3-Sulfopropyl)-2-vinylpyridiniumbetain und 2-Methacryloyloxyethylphosphorylcholin alleine oder als Mischung ausgewählt ist.

**8.** Dispersion nach einem der vorhergehenden Ansprüche, wobei das hydrophile kationische Monomer unter

- Dimethylaminopropyl(meth)acrylamid, Dimethylaminoethyl(meth)acrylamid,
- Diethylaminoethyl(meth)acrylat, Dimethylaminoethyl(meth)acrylat, Morpholinoethyl(meth)acrylat;
- Vinylimidazol, Vinylpyridin

und den nachstehenden Monomeren:

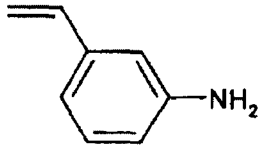

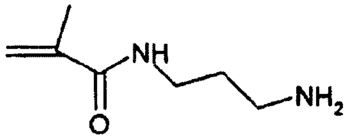

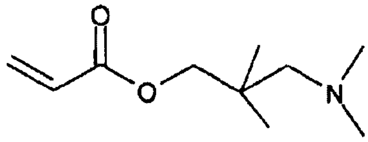 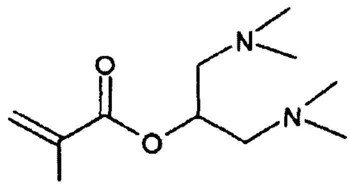

alleine oder als Mischung ausgewählt ist.

**9.** Dispersion nach einem der vorhergehenden Ansprüche, wobei das ethylenische Polymer 100 Gew.-% hydrophiles kationisches Monomer oder eine Mischung derartiger hydrophiler kationischer Monomere, bezogen auf das Gesamtgewicht der Startmonomere, umfasst.

**10.** Dispersion nach einem der Ansprüche 1 bis 8, wobei das ethylenische Polymer ein oder mehrere zusätzliche Monomere umfasst, die in einem Anteil von 0,01 bis 50 Gew.-%, bezogen auf das Gesamtgewicht der Monomere, vorliegen.

**11.** Dispersion nach einem der vorhergehenden Ansprüche, wobei das zusätzliche Monomer unter

- (i) (Meth)acrylsäureestern der Formel $CH_2$=CHCOOR4 oder $CH_2$=C($CH_3$)COOR4, wobei R4 für eine lineare, verzweigte oder cyclische, gesättigte oder ungesättigte auf Kohlenstoff basierende Kette mit 1 bis 32 Kohlenstoffatomen, die gegebenenfalls ein oder mehrere unter O, N und S ausgewählte eingeschobene Heteroatome umfasst und/oder gegebenenfalls durch einen oder mehrere unter -OH und Halogenatomen (Cl, Br, I und F) ausgewählte Substituenten substituiert ist, steht;
- (ii) (Meth)acrylsäureamiden der Formel $CH_2$=CHCONR$_5$R'$_5$ oder $CH_2$=C($CH_3$)CONR5R'5, worin R5 und R'5 gleich oder verschieden sind und für Wasserstoff oder eine lineare, verzweigte oder cyclische, gesättigte oder ungesättigte oder aromatische auf Kohlenstoff basierende Kette mit 6 bis 28 Kohlenstoffatomen, die gegebenenfalls ein oder mehrere unter O, N und S ausgewählte eingeschobene Heteroatome umfasst und/oder gegebenenfalls durch einen oder mehrere unter -OH und Halogenatomen (Cl, Br, I und F) ausgewählte Substituenten substituiert ist, stehen;
- (iii) Vinylestern der Formel $CH_2$=CH-OCO-R6, wobei R6 für eine lineare oder verzweigte, gesättigte oder ungesättigte auf Kohlenstoff basierende Kette mit 1 bis 12 Kohlenstoffatomen steht;
- (iv) Vinylethern der Formel $CH_2$=CHOR7, wobei R7 für eine lineare oder verzweigte, gesättigte oder ungesättigte auf Kohlenstoff basierende Kette mit 1 bis 12 Kohlenstoffatomen steht;
- (v) Vinylverbindungen der Formel $CH_2$=CHR8, worin R8 für
- eine Hydroxylgruppe;
- eine lineare oder verzweigte Alkylgruppe mit 1 bis 25 Kohlenstoffatomen, in die gegebenenfalls ein oder mehrere unter O, N, S und P ausgewählte Heteroatome eingeschoben sind; wobei die Alkylgruppe gegebenenfalls durch einen oder mehrere unter -OH und Halogenatomen (Cl, Br, I und F) ausgewählte Substituenten substituiert ist;
- eine $C_3$- bis $C_8$-Cycloalkylgruppe,
- eine $C_6$- bis $C_{20}$-Arylgruppe,
- eine $C_7$- bis $C_{30}$-Aralkylgruppe ($C_1$- bis $C_4$-Alkylgruppe),
- eine 4- bis 12-gliedrige heterocyclische Gruppe mit einem oder mehreren unter O, N und S ausgewählten Heteroatomen,
- eine Heterocycloalkylgruppe (Alkyl mit 1 bis 4 Kohlenstoffatomen)

steht, wobei die Cycloalkylgruppen, Arylgruppen, Aralkylgruppen, heterocyclischen Gruppen oder Heterocycloalkylgruppen gegebenenfalls durch einen oder mehrere unter der Hydroxylgruppe, Halogenatomen und linearen oder verzweigten $C_1$-$C_4$-Alkylgruppen ausgewählte Substituenten substituiert sind, in die gegebenenfalls ein oder mehrere unter O, N, S und P ausgewählte Heteroatome eingeschoben sind, wobei die Alkylgruppen gegebenenfalls durch einen oder mehrere unter -OH und Halogenatomen (Cl, Br, I und F) ausgewählte Substituenten substituiert sind;

- (vi) multifunktionellen Monomeren;
- (vii) (Meth)acrylsäure; Maleinsäureanhydrid; Crotonsäure, Itaconsäure, Fumarsäure, Maleinsäure, Diacrylsäure, Dimethylfumarsäure, Citraconsäure, Acrylamidopropansulfonsäure, 2-Acrylamido-2-methylpropansulfonsäure, Styrolsulfonsäure, Vinylbenzoesäure, Vinylphosphorsäure, Vinylsulfonsäure, Vinylbenzolsulfonsäure, Acrylamidoglykolsäure der Formel $CH_2$=CH-CONHCH(OH)COOH, Vinylphosphonsäure; Sulfopropyl(meth)acrylat, Sulfoethyl(meth)acrylat, Vinylmethylsulfon, 2-(Methacryloyloxy)ethylphosphat der Formel $CH_z=C(CH_3)COOC_2H_4OP(O)(OH)_2$ sowie Salzen davon und Mischungen davon;
- (viii) Poly(ethylenglykol)(meth)acrylaten, Alkylpoly(ethylenglykol)(meth)acrylaten und insbesondere Methylpoly(ethylenglykol)methacrylaten alleine oder als Mischung ausgewählt ist.

**12.** Dispersion nach Anspruch 11, wobei das zusätzliche Monomer unter Methyl-, Ethyl-, Propyl-, n-Butyl-, Isobutyl-, 2-Ethylhexyl-, tert.-Butyl-, Isooctyl-, Decyl-, Myristyl-, Stearyl(meth)acrylaten und -(meth)acrylamiden oder Styrol, Ethylhexylvinylether, Dodecylvinylether, Vinylhexanoat; 2-Hydroxyethylacrylat und Poly(ethylenglykol)methacrylat und Mischungen davon ausgewählt ist.

**13.** Dispersion nach einem der vorhergehenden Ansprüche, wobei die nichtwässrige flüssige Verbindung, die einen Gesamtlöslichkeitsparameter gemäß dem Löslichkeitsraum nach Hansen kleiner gleich 20 $(MPa)^{1/2}$ aufweist, unter natürlichen oder synthetischen, auf Kohlenstoff basierenden, auf Kohlenwasserstoff basierenden, fluorierten und/oder silikonisierten, gegebenenfalls verzweigten Ölen allein oder als Mischung ausgewählt ist.

**14.** Dispersion nach einem der vorhergehenden Ansprüche, wobei die nichtwässrige flüssige Verbindung unter:

- pflanzlichen Ölen aus Estern von Fettsäuren und Polyolen; Estern, die sich von langkettigen C6-C20-Säuren oder -Alkoholen ableiten; Estern der Formel RCOOR', worin R für den Rest einer höheren Fettsäure mit 7 bis 19 Kohlenstoffatomen steht und R' für eine auf Kohlenwasserstoff basierende Kette mit 3 bis 20 Kohlenstoffatomen steht;
- Kohlenwasserstoffen;
- Silikonölen, die gegebenenfalls durch gegebenenfalls fluorierte aliphatische und/oder aromatische Gruppen oder funktionelle Gruppen substituiert sind; und flüchtigen Silikonölen;
- Lösungsmitteln, allein oder als Mischung, die unter linearen, verzweigten oder cyclischen Estern mit 6 bis 30 Kohlenstoffatomen, Ethern mit 6 bis 30 Kohlenstoffatomen und Ketonen mit 6 bis 30 Kohlenstoffatomen ausgewählt sind;
- aliphatischen Fettmonoalkoholen mit mindestens 6 Kohlenstoffatomen, wobei die Kohlenwasserstoffkette keine Substituentengruppe umfasst; ausgewählt ist.

**15.** Dispersion nach einem der vorhergehenden Ansprüche, wobei die nichtwässrige flüssige Verbindung unter cyclischen oder linearen flüchtigen Silikonölen und/oder Estern der Formel RCOOR', worin R für den Rest einer höheren Fettsäure mit 7 bis 19 Kohlenstoffatomen steht und R' für eine auf Kohlenwasserstoff basierende Kette mit 3 bis 20 Kohlenstoffatomen steht; und Mischungen davon ausgewählt ist.

**16.** Dispersion nach einem der vorhergehenden Ansprüche, wobei das Stabilisierungsmittel unter Sequenzpolymeren, Pfropfpolymeren und/oder statistisch aufgebauten Polymeren allein oder als Mischung ausgewählt ist.

**17.** Dispersion nach einem der vorhergehenden Ansprüche, wobei das Stabilisierungsmittel in einem Anteil von 0,1 bis 30 Gew.-%, bezogen auf das Gewicht der Startmischung der Monomere vorliegt.

**18.** Dispersion nach einem der vorhergehenden Ansprüche, wobei das Stabilisierungsmittel unter:

- Silikonpolymeren, die mit einer Kohlenwasserstoffkette gepfropft sind, und Kohlenwasserstoffpolymeren, die mit einer Silikonkette gepfropft sind;
- Pfropfcopolymere mit unlöslicher Hauptkette vom Polyacryltyp mit löslichen Pfropfanteilen vom Typ Poly(12-hydroxystearinsäure);
- Pfropf- oder Sequenzblockcopolymeren mit mindestens einem Block vom Polyorganosiloxan-Typ und mindestens einem Block eines (i) aus einer Radikalpolymerisation oder (ii) aus einer Polykondensation hervorgegangenen Polymers oder eine Mischung davon, wobei das Copolymer fluorierte Einheiten enthalten kann;
- Pfropf- oder Sequenzblockcopolymeren von C1-C4-Alkyl(meth)acrylaten und C8-C30-Alkyl(meth)acrylaten;
- Pfropf- oder Sequenzblockcopolymeren mit mindestens einem Block aus der Polymerisation von ethylenischem Monomer mit einer oder mehreren ethylenischen Bindungen, die gegebenenfalls konjugiert sind, und mindestens einem aus der Radikalpolymerisation außer Dienen hervorgegangenen Polymerblock;
- Alkyldimethiconen, worin die Alkylgruppe 6 bis 32 Kohlenstoffatome umfasst;
- Dimethiconolester der Formel:

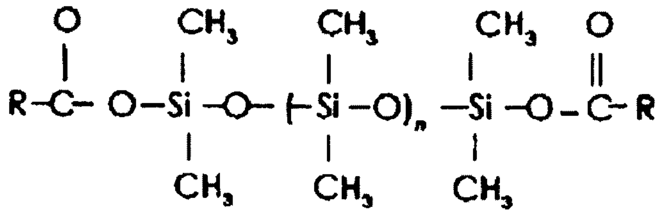

worin R für einen Alkylrest mit 6 bis 32 Kohlenstoffatomen steht,
- Alkylamidoaminen mit insbesondere 6 bis 60 Kohlenstoffatomen;
- Copolymeren mit mindstens einem Polyorganosiloxanteil und fluorierten Gruppen, insbesondere der Formel:

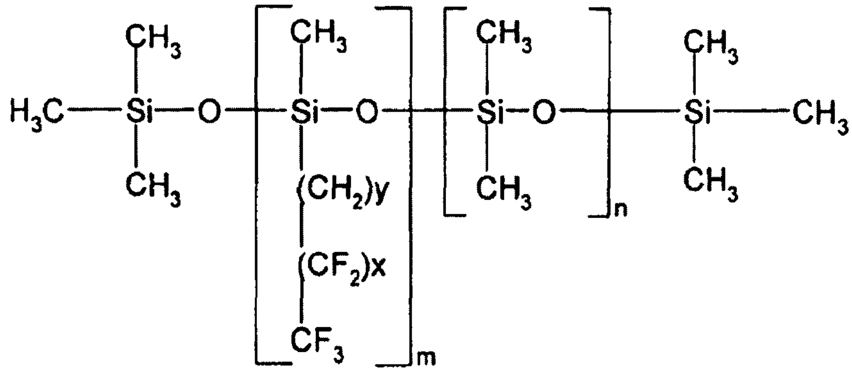

worin x für eine ganze Zahl zwischen 3 und 12 steht; y für eine ganze Zahl zwischen 2 und 6 steht und m und n so beschaffen sind, dass das Molekulargewicht der Verbindung zwischen 5000 und 15.000 liegt;
ausgewählt ist.

**19.** Dispersion nach einem der vorhergehenden Ansprüche, wobei das Stabilisierungsmittel unter:

- fluorierten Silikonen oder Fluorsilikonen der Formel:

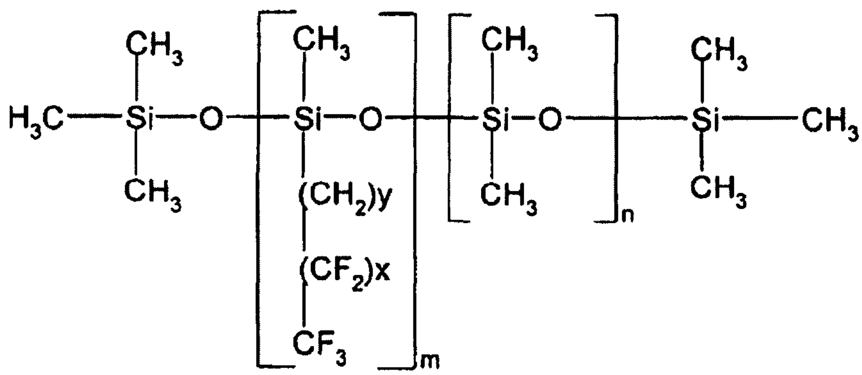

worin x = 8, y = 2 oder 3 und m und n so beschaffen sind, dass das Molekulargewicht der Verbindung zwischen 5000 und 15.000 liegt; und
- Pfropf- oder Sequenzblockcopolymeren mit mindestens einem Block vom Polyorganosiloxan-Typ und mindestens einem Block eines aus einer Polykondensation hervorgegangenen Polymers, insbesondere vom Polyether-, Polyester- oder Polyamid-Typ; insbesondere einem Polyetherblock vom $C_2$-$C_{18}$-Polyoxyalkylen-Typ, insbesondere vom Polyoxyethylen- und/oder Polyoxypropylen-Typ; ausgewählt ist.

**20.** Kosmetische oder pharmazeutische Zusammensetzung, umfassend mindestens eine Dispersion gemäß einem der Ansprüche 1 bis 19 in einem kosmetisch oder pharmazeutisch unbedenklichen Medium, das mindestens einen

unter Wachsen, Ölen, Gummen und/oder pastösen Fettkörpern tierischen, pflanzlichen, mineralischen oder synthetischen oder sogar silikonischen Ursprungs und Mischungen davon; einem oder mehreren farbgebenden Stoffen, die unter pulverförmigen Verbindungen und/oder fettlöslichen oder wasserlöslichen Farbstoffen ausgewählt sind; Antioxidantien, Parfümen, etherischen Ölen, Konservierungsmitteln, kosmetischen Wirkstoffen, Feuchtigkeitsspendern, Vitaminen, essentiellen Fettsäuren, Ceramiden, Sonnenschutzmitteln, Tensiden, Polymeren, Verdickungsmitteln und Geliermitteln ausgewählten Bestandteil umfasst.

**21.** Zusammensetzung nach Anspruch 20, die in Form einer Suspension, einer Dispersion; einer gegebenenfalls verdickten oder sogar gelierten öligen Lösung; einer Öl-in-Wasser-Emulsion, Wasser-in-Öl-Emulsion oder multiplen Emulsion; eines Gels oder eines Schaums; eines öligen oder emulgierten Gels; einer Dispersion von Vesikeln; einer zweiphasigen oder multiphasigen Lotion; eines Sprays; einer Lotion, einer Creme, einer Pomade; einer weichen Paste, einer Salbe, eines gegossenen oder geformten Feststoffs oder eines kompaktierten Feststoffs vorliegt.

**22.** Zusammensetzung nach einem der Ansprüche 20 bis 21, die in Form eines Produkts zur Pflege und/oder zum Make-up der Körper- oder Gesichtshaut, der Lippen und der Haare, eines Sonnenschutz- oder Selbstbräunungsprodukts oder eines Haarprodukts vorliegt.

**23.** Zusammensetzung nach einem der Ansprüche 20 bis 22, die in Form eines Shampoos, eines Gels, einer Lotion für die Wasserwelle, einer Lotion für die Fönwelle, einer Zusammensetzung zur Fixierung der Frisur wie Lacken, Schäumen oder Sprays; eines Duschgels, eines Schaumbads; einer Wash-out- oder Leave-in-Haarpflegespülung, einer Dauerwellen-, Entkrausungs-, Färbe- oder Bleichzusammensetzung; einer Wash-out-Zusammensetzung zur Anwendung vor oder nach einer Färbung, Bleichung, Dauerwelle oder Entkrausung oder auch zwischen zwei Schritten einer Dauerwelle oder Entkrausung; in Form von Waschzusammensetzungen für die Haut und insbesondere in Form von Lösungen oder Gelen für Bad oder Dusche oder Abschminkprodukten; wässrigen oder wässrig-alkoholischen Lotionen für die Pflege der Haut und/oder der Haare oder eines Haarpflegeprodukts (Haarmaske) vorliegt.

**24.** Verfahren zur kosmetischen Behandlung von Keratinmaterialien, bei dem man auf die Materialien eine kosmetische Zusammensetzung gemäß einem der Ansprüche 20 bis 23 aufbringt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1323753 A
- WO 9115185 A
- WO 9818433 A
- EP 749747 A


### Littérature non-brevet citée dans la description


- **BRANDRUP ; IMMERGUT ; GRULKE.** Polymer Handbook. John Wiley, 1999
- Solubility parameter values. **GRULKE.** Polymer Handbook. 519-559
- **HANSEN.** The three dimensionnal solubility parameters. *J. Paint Technol.,* 1967, vol. 39, 105